# EUROPEAN PATENT APPLICATION

(11) **EP 4 838 223 A1**
(43) Date of publication of application: **11.11.2026**
(21) Application number: 23962866.2
(22) Date of filing: 29.12.2023
(51) Int. Cl.: G01N 33/543

(54) **SAMPLE ANALYZER, SAMPLE ANALYSIS METHOD, AND REAGENT**

(71) Applicant: Shenzhen Mindray Bio-Medical Electronics Co., Ltd., Shenzhen, Guangdong 518057 (CN)
(72) Inventor: HUANG, Bin, Shenzhen, Guangdong 518057 (CN); JIANG, Ming, Shenzhen, Guangdong 518057 (CN); WANG, Lu, Shenzhen, Guangdong 518057 (CN)
(74) Representative: KIPA AB
(86) International application number: PCT/CN2023/143509
(87) International publication number: WO 2025/138192

(57) **Abstract**

The present invention relates to a sample analyzer, a sample analysis method, and a reagent kit. A controller in the analyzer is configured to perform the following operations: controlling a reagent dispensing mechanism to suction a first reagent and a sample under test and provide same into a reaction cup to obtain a first reaction system for reaction for a first predetermine time, wherein the first reagent comprises a first carrier having a first ligand bound to the surface thereof, wherein the first ligand can bind to an analyte; controlling the reagent dispensing mechanism to further suction a second reagent and provide same into the reaction cup to obtain a second reaction system for reaction for a second predetermined time, wherein the second reagent comprises a second ligand that can bind to the analyte; controlling an optical measurement component to acquire at least two optical signals, so as to obtain a first reaction analysis value of the first reaction system and a second reaction analysis value of the second reaction system; and obtaining an analysis result of the analyte on the basis of the two reaction analysis values.

## Description

### Technical Field

The present application relates to the field of in vitro analysis, and particularly to a sample analyzer, a sample analysis method, and a corresponding reagent.

### Background Art

Immunoturbidimetry is an important method in clinical biochemical testing, which utilizes the affinity interaction (e.g., antibody-antigen interaction) between the analyte (e.g., protein) and its ligand to detect the concentration of the analyte in a sample. The use of ligand-coated latex has been proposed to enhance the sensitivity and accuracy of detection.

The detection limit of latex-enhanced immunoturbidimetry is strongly correlated with the particle size of the latex microspheres used. Generally, latex microspheres with higher light scattering (larger size) have lower detection lower limits but also lower detection upper limits; conversely, latex microspheres with weaker light scattering (smaller size) have higher detection lower limits and higher detection upper limits.

However, in clinical testing, many analytes such as C-reactive protein (CRP), urinary microalbumin (MALB), rheumatoid factor (RF), and β2-microglobulin (β2-MG) have wide concentration ranges. Therefore, there has been a need to expand the linear range of related detection.

For example, a method has been proposed to mix two types of latex microspheres with different light scattering properties to achieve a wide linear detection range. This method is based on the principle that different microsphere sizes have different detection ranges. When the analyte concentration is low, the microspheres with strong light scattering properties bind to the analyte, achieving a lower detection lower limit; when the analyte concentration is high, the microspheres with weak light scattering properties bind to the analyte, achieving a higher detection upper limit. However, in practical detection, both types of microspheres coexist within the detection system, making it difficult for them to play a dominant role respectively as desired when the concentrations of the analytes vary. For example, when the analyte concentration in the sample is low, both types of microspheres can bind to the analyte, wasting the opportunity for the microspheres with higher light scattering properties to bind, resulting in a signal that fails to meet detection requirements. Therefore, the latex-enhanced immunoturbidimetry still needs further improvement.

### Summary

The purpose of the present disclosure is to provide a sample analyzer, a sample analysis method, and a reagent. The analyzer and method avoid the problems caused by mixing latex microspheres with different light scattering properties, further expanding the linear detection range.

Therefore, in a first aspect, in one embodiment, the present disclosure provides a sample analyzer, comprising a sample component, a reagent component, a measurement component, and a controller.

The sample component comprises a sample-carrying component for carrying a sample to be tested and a sample-dispensing mechanism.

The reagent component comprises a reagent-carrying component for carrying a reagent and a reagent-dispensing mechanism.

The measurement component comprises a reaction component and an optical measurement component, wherein the reaction component has at least one placement position for placing a reaction cup.

The controller is configured to perform the following operations:
controlling the reagent-dispensing mechanism to draw a first reagent and dispense it into the reaction cup, wherein the first reagent comprises a first carrier, the surface of which is bound with a first ligand capable of binding to an analyte in the sample to be tested; controlling the sample-dispensing mechanism to draw the sample to be tested and dispense it into the reaction cup to obtain a first reaction system; controlling the first reaction system to react for a first predetermined time;
controlling the reagent-dispensing mechanism to further draw a second reagent and dispense it into the reaction cup to obtain a second reaction system, wherein the second reagent comprises a second ligand capable of binding to the analyte; controlling the second reaction system to react for a second predetermined time;
controlling the optical measurement component to acquire at least two optical signals;
obtaining a first reaction analysis value of the first reaction system based on at least one of the optical signals, and obtaining a second reaction analysis value of the second reaction system based on at least another of the optical signals; and
performing a determination based on the first reaction analysis value and/or the second reaction analysis value, and selecting one of the first reaction analysis value and the second reaction analysis value according to the determination result to obtain an analysis result of the analyte in the sample to be tested, or, selecting the optical signal corresponding to one of the first reaction analysis value and the second reaction analysis value according to the determination result to obtain an analysis result of the analyte in the sample to be tested.

In another embodiment of the first aspect, the present disclosure provides a sample analyzer, comprising a sample component, a reagent component, a measurement component, and a controller.

The sample component comprises a sample-carrying component for carrying a sample to be tested and a sample-dispensing mechanism.

The reagent component comprises a reagent-carrying component for carrying a reagent and a reagent-dispensing mechanism.

The measurement component comprises a reaction component and an optical measurement component, wherein the reaction component has at least one placement position for placing a reaction cup.

The controller is configured to perform the following operations:
controlling the reagent-dispensing mechanism to draw a first reagent and dispense it into the reaction cup, wherein the first reagent comprises a first microsphere, the surface of which is bound with a first ligand capable of binding to an analyte in the sample to be tested; controlling the sample-dispensing mechanism to draw the sample to be tested and dispense it into the reaction cup to obtain a first reaction system; controlling the first reaction system to react for a first predetermined time;
controlling the reagent-dispensing mechanism to further draw a second reagent and dispense it into the reaction cup to obtain a second reaction system, wherein the second reagent comprises a second ligand capable of binding to the analyte; controlling the second reaction system to react for a second predetermined time;
controlling the optical measurement component to acquire at least two optical signals;
obtaining a first reaction analysis value of the first reaction system based on at least one of the optical signals, and obtaining a second reaction analysis value of the second reaction system based on at least another of the optical signals; and
obtaining an analysis result of the analyte in the sample to be tested based on the summation result of the first reaction analysis value and the second reaction analysis value.

In some embodiments, controlling the optical measurement component to acquire at least two optical signals, comprises:
acquiring at least one of the optical signals after controlling the reagent-dispensing mechanism to draw the first reagent and dispense it into the reaction cup and before controlling the reagent-dispensing mechanism to further draw the second reagent and dispense it into the reaction cup; and acquiring at least another of the optical signals after controlling the reagent-dispensing mechanism to further draw the second reagent and dispense it into the reaction cup; or,
acquiring the at least two optical signals after controlling the reagent-dispensing mechanism to further draw the second reagent and dispense it into the reaction cup.

In some embodiments, performing a determination based on the first reaction analysis value and/or the second reaction analysis value, and selecting one of the first reaction analysis value and the second reaction analysis value according to the determination result to obtain an analysis result of the analyte in the sample to be tested, or, selecting the optical signal corresponding to one of the first reaction analysis value and the second reaction analysis value according to the determination result to obtain an analysis result of the analyte in the sample to be tested, comprises:
comparing the first reaction analysis value and the second reaction analysis value, and selecting one of the first reaction analysis value and the second reaction analysis value according to the comparison result to obtain the analysis result of the analyte in the sample to be tested, or, selecting the optical signal corresponding to one of the first reaction analysis value and the second reaction analysis value according to the comparison result to obtain the analysis result of the analyte in the sample to be tested;
preferably, comparing the first reaction analysis value and the second reaction analysis value, and selecting the larger one of the first reaction analysis value and the second reaction analysis value according to the comparison result to obtain the analysis result of the analyte in the sample to be tested, or selecting the optical signal corresponding to the larger one of the first reaction analysis value and the second reaction analysis value according to the comparison result to obtain the analysis result of the analyte in the sample to be tested.

In some embodiments, performing a determination judgment based on the first reaction analysis value and/or the second reaction analysis value, and selecting one of the first reaction analysis value and the second reaction analysis value according to the determination result to obtain an analysis result of the analyte in the sample to be tested, or, selecting the optical signal corresponding to one of the first reaction analysis value and the second reaction analysis value according to the determination result to obtain an analysis result of the analyte in the sample to be tested, comprises:
determining whether the first reaction analysis value and/or the second reaction analysis value are within a preset range, and selecting one of the first reaction analysis value and the second reaction analysis value according to the determination result to obtain the analysis result of the analyte in the sample to be tested, or selecting the optical signal corresponding to one of the first reaction analysis value and the second reaction analysis value according to the determination result to obtain the analysis result of the analyte in the sample to be tested.

In some embodiments, controlling the optical measurement component to acquire at least two optical signals, comprises: acquiring at least one of the optical signals at a first detection wavelength, and acquiring at least another of the optical signals at a second detection wavelength. Preferably, the first detection wavelength and the second detection wavelength are different. Preferably, the first detection wavelength and the second detection wavelength are selected from 320nm to 860nm; more preferably, the first detection wavelength and the second detection wavelength are selected from 340nm to 850nm.

In some embodiments, controlling the optical measurement component to acquire at least two optical signals, comprises:
acquiring a first optical signal after controlling the reagent-dispensing mechanism to draw the first reagent and dispense it to the reaction cup; or, acquiring a first optical signal after controlling the reagent-dispensing mechanism to draw the first reagent and dispense it to the reaction cup and controlling the sample-dispensing mechanism to draw the sample to be tested and dispense it to the reaction cup and before the start of the first predetermined time; acquiring a second optical signal after the end of the first predetermined time and before controlling the reagent-dispensing mechanism to further draw the second reagent and dispense it to the reaction cup; acquiring a third optical signal after controlling the reagent-dispensing mechanism to further draw the second reagent and dispense it to the reaction cup and before the start of the second predetermined time; and, acquiring a fourth optical signal after the end of the second predetermined time.

Obtaining a first reaction analysis value of the first reaction system based on at least one of the optical signals, and obtaining a second reaction analysis value of the second reaction system based on at least another of the optical signals, comprises: obtaining the first reaction analysis value of the first reaction system based on the first optical signal and the second optical signal; and, obtaining the second reaction analysis value of the second reaction system based on the third optical signal and the fourth optical signal.

In some embodiments, controlling the optical measurement component to acquire at least two optical signals, comprises: acquiring the second optical signal after the end of the first predetermined time and before controlling the reagent-dispensing mechanism to further draw the second reagent and dispense it into the reaction cup; and acquiring the fourth optical signal after the end of the second predetermined time.

Obtaining the first reaction analysis value of the first reaction system based on at least one of the optical signals, and obtaining the second reaction analysis value of the second reaction system based on at least another of the optical signals, comprises: obtaining the first reaction analysis value of the first reaction system based on the second optical signal, and obtaining the second reaction analysis value of the second reaction system based on the fourth optical signal or based on both the fourth optical signal and the second optical signal.

In some embodiments, controlling the optical measurement component to acquire at least two optical signals, comprises: acquiring a third optical signal after controlling the reagent-dispensing mechanism to further draw the second reagent and dispensing it into the reaction cup and before the start of the second predetermined time; and acquiring a fourth optical signal after the end of the second predetermined time.

Obtaining the first reaction analysis value of the first reaction system based on at least one of the optical signals, and obtaining the second reaction analysis value of the second reaction system based on at least another of the optical signals, comprises: obtaining the first reaction analysis value of the first reaction system based on the third optical signal, and obtaining the second reaction analysis value of the second reaction system based on the fourth optical signal or based on both the fourth optical signal and the third optical signal.

In some embodiments, controlling the optical measurement component to acquire at least two optical signals, comprises: acquiring a first optical signal after controlling the reagent-dispensing mechanism to draw the first reagent and dispense it into the reaction cup; or, acquiring a first optical signal after controlling the reagent-dispensing mechanism to draw the first reagent and dispense it into the reaction cup and controlling the sample-dispensing mechanism to draw the sample to be tested and dispense it into the reaction cup and before the start of the first predetermined time; acquiring a second optical signal after the end of the first predetermined time and before controlling the reagent-dispensing mechanism to further draw the second reagent and dispense it into the reaction cup; and acquiring a fourth optical signal after the end of the second predetermined time.

Obtaining the first reaction analysis value of the first reaction system based on at least one of the optical signals, and obtaining the second reaction analysis value of the second reaction system based on at least another of the optical signals, comprises: obtaining the first reaction analysis value of the first reaction system based on both the first optical signal and the second optical signal, and obtaining the second reaction analysis value of the second reaction system based on both the first optical signal and the fourth optical signal, or based on both the second optical signal and the fourth optical signal.

In some embodiments, controlling the optical measurement component to acquire at least two optical signals, comprises: collecting multiple optical signals over at least a period from the start of the first predetermined time to the end of the first predetermined time to acquire a first reaction curve; and, collecting multiple optical signals over at least a period from the start of the second predetermined time to the end of the second predetermined time to acquire a second reaction curve.

Obtaining the first reaction analysis value of the first reaction system based on at least one of the optical signals, and obtaining the second reaction analysis value of the second reaction system based on at least another of the optical signals, comprises: obtaining the first reaction analysis value of the first reaction system based on the first reaction curve, and obtaining the second reaction analysis value of the second reaction system based on the second reaction curve;
preferably, obtaining the first reaction analysis value of the first reaction system based on the first reaction curve or based on a characteristic value of the first reaction curve, and obtaining the second reaction analysis value of the second reaction system based on the second reaction curve or based on a characteristic value of the second reaction curve.

In another embodiment of the first aspect, the present disclosure provides a sample analyzer, comprising: a sample component, a reagent component, a measurement component, and a controller.

The sample component comprises a sample-carrying component for carrying a sample to be tested and a sample-dispensing mechanism.

The reagent component comprises a reagent-carrying component for carrying a reagent and a reagent-dispensing mechanism.

The measurement component comprises a reaction component and an optical measurement component, wherein the reaction component has at least one placement position for placing a reaction cup.

The controller is configured to perform the following operations:
controlling the reagent-dispensing mechanism to draw a first reagent and dispense it into the reaction cup, wherein the first reagent comprises a first carrier, the surface of which is bound with a first ligand capable of binding to an analyte in the sample to be tested; controlling the sample-dispensing mechanism to draw the sample to be tested and dispense it into the reaction cup to obtain a first reaction system; controlling the first reaction system to react for a first predetermined time;
controlling the optical measurement component to acquire at least one of the optical signals;
obtaining a first reaction analysis value of the first reaction system based on the at least one of the optical signals;
performing a determination based on the first reaction analysis value.

When the determination result meets a preset condition, an analysis result of the analyte in the sample to be tested is obtained based on the first reaction analysis value.

When the determination result does not meet the preset condition, the reagent-dispensing mechanism is controlled to further draw a second reagent and dispense it into the reaction cup to obtain a second reaction system, wherein the second reagent comprises a second ligand capable of binding to the analyte, and the reaction system is controlled to react for a second predetermined time;
the optical measurement component is controlled to acquire at least another of the optical signals;
a second reaction analysis value of the second reaction system is obtained based at least on the at least another of the optical signals; and
an analysis result of the analyte in the sample to be tested is obtained based at least on the second reaction analysis value.

In some embodiments, the determination based on the first reaction analysis value comprises determining whether the first reaction analysis value is within the preset range;
if so, obtaining the analysis result of the analyte in the sample to be tested based on the first reaction analysis value or the optical signal corresponding to the first reaction analysis value;
if not, controlling the reagent-dispensing mechanism to further draw a second reagent and dispense it to the reaction cup to obtain a second reaction system, wherein the second reagent comprises a second ligand capable of binding to the analyte; controlling the second reaction system to react for a second predetermined time;
controlling the optical measurement component to acquire the at least another of the optical signals;
obtaining a second reaction analysis value of the second reaction system based at least on the at least another of the optical signals; and
obtaining an analysis result of the analyte in the sample to be tested based at least on the second reaction analysis value.

In some embodiments, obtaining the analysis result of the analyte in the sample to be tested based at least on the second reaction analysis value comprises: obtaining the analysis result of the analyte in the sample to be tested based on the first reaction analysis value and the second reaction analysis value.

In some embodiments, obtaining the analysis result of the analyte in the sample to be tested based at least on the first reaction analysis value and the second reaction analysis value comprises: preforming a determination based on the first reaction analysis value and/or the second reaction analysis value, and selecting one of the first reaction analysis value and the second reaction analysis value according to the determination result to obtain the analysis result of the analyte in the sample to be tested; or, selecting the optical signal corresponding to one of the first reaction analysis value and the second reaction analysis value according to the determination result to obtain the analysis result of the analyte in the sample to be tested.

In some embodiments, obtaining the analysis result of the analyte in the sample to be tested based on the first reaction analysis value and the second reaction analysis value comprises: obtaining the analysis result of the analyte in the sample to be tested based on the summation result of the first reaction analysis value and the second reaction analysis value.

In some embodiments, the optical signal comprises a scattered optical signal and/or a transmitted optical signal.

In a second aspect, in one embodiment, the present disclosure provides a sample analysis method. The method comprises the following steps:
controlling a sample to be tested to mix with a first reagent to obtain a first reaction system and allowing the first reaction system to react for a first predetermined time, wherein the first reagent comprises a first carrier, the surface of which is bound with a first ligand capable of binding to an analyte in the sample to be tested;
controlling a second reagent to mix with the first reaction system to obtain a second reaction system and allowing the second reaction system to react for a second predetermined time, wherein the second reagent comprises a second ligand capable of binding to the analyte;
acquiring at least two optical signals;
obtaining a first reaction analysis value of the first reaction system based on at least one of the optical signals, and obtaining a second reaction analysis value of the second reaction system based on at least another of the optical signals;
performing a determination based on the first reaction analysis value and/or the second reaction analysis value, and selecting one of the first reaction analysis value and the second reaction analysis value according to the determination result to obtain an analysis result of the analyte in the sample to be tested, or selecting the optical signal corresponding to one of the first reaction analysis value and the second reaction analysis value according to the determination result to obtain an analysis result of the analyte in the sample to be tested.

In another embodiment of the second aspect, the present disclosure provides a sample analysis method, the method comprising the following steps:
controlling a sample to be tested to mix with a first reagent to obtain a first reaction system and allowing the first reaction system to react for a first predetermined time, wherein the first reagent comprises a first carrier, the surface of which is bound with a first ligand capable of binding to an analyte in the sample to be tested;
controlling a second reagent to mix with the first reaction system to obtain a second reaction system and allowing the second reaction system to react for a second predetermined time, wherein the second reagent comprises a second ligand capable of binding to the analyte;
acquiring at least two optical signals;
obtaining a first reaction analysis value of the first reaction system based on at least one of the optical signals, and obtaining a second reaction analysis value of the second reaction system based on at least another of the optical signals; and
obtaining an analysis result of the analyte in the sample to be tested based on the summation result of the first reaction analysis value and the second reaction analysis value.

In some embodiments, acquiring at least two optical signals comprises: acquiring at least one of the optical signals after controlling the sample to be tested to mix with the first reagent and before controlling the second reagent to mix with the first reaction system, and acquiring at least another of the optical signals after controlling the second reagent to mix with the first reaction system; or, acquiring the at least two optical signals after controlling the second reagent to mix with the first reaction system.

In some embodiments, performing a determination based on the first reaction analysis value and/or the second reaction analysis value, and selecting one of the first reaction analysis value and the second reaction analysis value according to the determination result to obtain an analysis result of the analyte in the sample to be tested, or selecting the optical signal corresponding to one of the first reaction analysis value and the second reaction analysis value according to the determination result to obtain an analysis result of the analyte in the sample to be tested, comprises:
comparing the first reaction analysis value and the second reaction analysis value, and selecting one of the first reaction analysis value and the second reaction analysis value according to the comparison result to obtain the analysis result of the analyte in the sample to be tested, or, selecting the optical signal corresponding to one of the first reaction analysis value and the second reaction analysis value according to the comparison result to obtain the analysis result of the analyte in the sample to be tested;
preferably, comparing the first reaction analysis value and the second reaction analysis value, and selecting the larger one of the first reaction analysis value and the second reaction analysis value according to the comparison result to obtain the analysis result of the analyte in the sample to be tested, or selecting the optical signal corresponding to the larger one of the first reaction analysis value and the second reaction analysis value according to the comparison result to obtain the analysis result of the analyte in the sample to be tested.

In some embodiments, performing a determination based on the first reaction analysis value and/or the second reaction analysis value, and selecting one of the first reaction analysis value and the second reaction analysis value according to the determination result to obtain an analysis result of the analyte in the sample to be tested, or selecting the optical signal corresponding to one of the first reaction analysis value and the second reaction analysis value according to the determination result to obtain an analysis result of the analyte in the sample to be tested, comprises:
determining whether the first reaction analysis value or the second reaction analysis value is within a preset range, and selecting one of the first reaction analysis value and the second reaction analysis value according to the determination result to obtain the analysis result of the analyte in the sample to be tested, or selecting the optical signal corresponding to one of the first reaction analysis value and the second reaction analysis value according to the determination result to obtain the analysis result of the analyte in the sample to be tested.

In some embodiments, acquiring at least two optical signals comprises: acquiring at least one of the optical signals at a first detection wavelength, and acquiring at least another of the optical signals at a second detection wavelength. Preferably, the first detection wavelength and the second detection wavelength are not the same. Preferably, the first detection wavelength and the second detection wavelength are selected from 320nm to 860nm; more preferably, the first detection wavelength and the second detection wavelength are selected from 340nm to 850nm.

In some embodiments, acquiring at least two optical signals comprises: acquiring a first optical signal based on the first reagent, or acquiring a first optical signal after controlling the sample to be tested to mix with the first reagent and before the start of the first predetermined time; acquiring a second optical signal after the end of the first predetermined time and before controlling the second reagent to mix with the first reaction system; acquiring a third optical signal after controlling the second reagent to mix with the first reaction system and before the start of the second predetermined time; and acquiring a fourth optical signal after the end of the second predetermined time.

Obtaining a first reaction analysis value of the first reaction system based on at least one of the optical signals and obtaining a second reaction analysis value of the second reaction system based on at least another of the optical signals comprises: obtaining a first reaction analysis value of the first reaction system based on the first optical signal and the second optical signal, and obtaining a second reaction analysis value of the second reaction system based on the third optical signal and the fourth optical signal.

In some embodiments, acquiring at least two optical signals comprises: acquiring a second optical signal after the end of the first predetermined time and before controlling the second reagent to mix with the first reaction system; and acquiring a fourth optical signal after the end of the second predetermined time.

Obtaining a first reaction analysis value of the first reaction system based on at least one of the optical signals, and obtaining a second reaction analysis value of the second reaction system based on at least another of the optical signals, comprises: obtaining a first reaction analysis value of the first reaction system based on the second optical signal, and obtaining a second reaction analysis value of the second reaction system based on the fourth optical signal or based on the fourth optical signal and the second optical signal.

In some embodiments, obtaining at least two optical signals comprises: obtaining a third optical signal after controlling the second reagent to mix with the first reaction system and before the start of the second predetermined time; and obtaining a fourth optical signal after the end of the second predetermined time.

Obtaining a first reaction analysis value of the first reaction system based on at least one of the optical signals, and obtaining a second reaction analysis value of the second reaction system based on at least another of the optical signals, comprises: obtaining a first reaction analysis value of the first reaction system based on the third optical signal, and obtaining a second reaction analysis value of the second reaction system based on the fourth optical signal or based on the fourth optical signal and the third optical signal.

In some embodiments, acquiring at least two optical signals comprises: acquiring a first optical signal based on the first reagent, or acquiring a first optical signal after controlling the sample to be tested to mix with the first reagent and before the start of the first predetermined time; acquiring a second optical signal after the end of the first predetermined time and before controlling the second reagent to mix with the first reaction system; and acquiring a fourth optical signal after the end of the second predetermined time.

Obtaining a first reaction analysis value of the first reaction system based on at least one of the optical signals, and obtaining a second reaction analysis value of the second reaction system based on at least another of the optical signals, comprises: obtaining a first reaction analysis value of the first reaction system based on the first optical signal and the second optical signal, and obtaining a second reaction analysis value of the second reaction system based on the first optical signal and the fourth optical signal, or based on the second optical signal and the fourth optical signal.

In some embodiments, acquiring at least two optical signals comprises: continuously collecting the optical signals for at least a period from the start of the first predetermined time to the end of the first predetermined time, and acquiring a first reaction curve; and continuously collecting the optical signals for at least a period from the start of the second predetermined time to the end of the second predetermined time, thereby acquiring a second reaction curve.

Obtaining a first reaction analysis value of the first reaction system based on at least one of the optical signals, and obtaining a second reaction analysis value of the second reaction system based on at least another of the optical signals, comprises: obtaining a first reaction analysis value of the first reaction system based on the first reaction curve, and obtaining a first reaction analysis value of the first reaction system based on the second reaction curve; preferably, obtaining the first reaction analysis value of the first reaction system based on the first reaction curve or based on a characteristic value of the first reaction curve, and obtaining the first reaction analysis value of the first reaction system based on the second reaction curve or based on a characteristic value of the second reaction curve.

In some embodiments, the binding affinity of the first ligand to the analyte is higher than that of the second ligand to the analyte.

In some embodiments, the second ligand in the second reagent exists in a free form, and/or in a form bound to the surface of the second carrier.

In some embodiments, the first carrier in the first reagent comprises latex microspheres having a first particle size, and the second carrier in the second reagent comprises latex microspheres having a second particle size, in which the second particle size is smaller than the first particle size.

In some embodiments, the first ligand and the second ligand are antigens or antibodies capable of binding to the analyte.

In some embodiments, the optical signal comprises a scattered optical signal and/or a transmitted optical signal.

A third aspect of the present disclosure provides a kit, comprising a first reagent and a second reagent. The first reagent comprises a first carrier, the surface of which is bound with a first ligand. The second reagent comprises a second ligand in free form. The first ligand and the second ligand may be the same or different and can bind to the same analyte in the same sample.

In some embodiments, the first carrier comprises first latex microspheres, and the first microspheres have a particle size of 40 nm to 500 nm, preferably 80 nm to 400 nm.

In some embodiments, the first ligand and the second ligand are antigens or antibodies capable of binding to the analyte in the sample to be tested.

In some embodiments, the binding affinity of the first ligand to the analyte is higher than the binding affinity of the second ligand to the analyte.

In some embodiments, the first reagent further comprises a first buffer and optionally an anti-interference agent; and the second reagent further comprises a second buffer.

In some embodiments, the reagent further comprises a pretreatment reagent, and the pretreatment reagent comprises a third buffer and optionally an anti-interference agent.

The analyzer and method as described herein use two reagents, one with a lower detection lower limit and the other with a higher detection upper limit, which are added sequentially to a sample to be tested, and the concentration of an analyte in the sample is obtained by utilizing the optical signals generated from the two stages of the reaction between the sample and the two reagents. This avoids the problem of suboptimal reaction order between ligands coated on different light-scattering particles and the analyte caused by mixing two reagents. Furthermore, by fully utilizing the signals from both stages of the reaction, more accurate results can be obtained, and the linear detection range can be further extended. In addition, since the two reagents are added sequentially to the sample to be tested, and multiple optical signals from both stages can be acquired during this process, a more flexible and diverse detection method is available to adapt to the needs of different detection scenarios.

### Brief Description of the Drawings

In the accompanying drawing, identical reference signs denote identical or similar components.
FIG. 1 shows a schematic flowchart of a sample analysis method according to one example.
FIG. 2 shows a schematic flowchart of a sample analysis method according to another example.
FIG. 3 shows a schematic diagram of the components of a sample analyzer according to one example.
FIG. 4 shows a schematic diagram of the components of a sample analyzer according to another example.
FIG. 5 shows a schematic diagram of the structure of some components of a sample analyzer according to one example.

### Detailed Description

The technical solution of the present invention will be clearly and completely described below with reference to specific examples and accompanying drawings. Obviously, the described examples are only a part of the embodiments of the present invention, not all of them. All other embodiments obtained by those skilled in the art based on the embodiments of the present invention without creative effort are within the scope of protection of the present invention.

Throughout this specification, unless otherwise specified, the terms used herein should be understood as having the meanings commonly used in the art. Therefore, unless otherwise defined, all technical and scientific terms used herein have the same meaning as generally understood by those skilled in the art to which the present invention pertains. In case of any conflict, this specification takes precedence.

In the present disclosure, the steps of the method are described in a certain order, or different steps are indicated by numbers in the accompanying drawings. It should be understood that the order in which the steps are described, or the magnitude of the numbers indicating the steps, is not intended to limit the actual order in which the steps of the method are performed. That is, the steps may be performed in the described order or in ascending order of the reference numerals; or they may not be performed in the described order or in ascending order of the reference numerals. For example, a step described later may be performed before a step described earlier; or two or more steps may be performed simultaneously.

As mentioned above, to broaden the detection range of latex-enhanced immunoturbidimetry, a method of mixing two latex microspheres with different light scattering properties in the detection reagent has been proposed. Generally, larger microspheres are beneficial for extending the detection lower limit, while smaller microspheres are beneficial for extending the detection upper limit. Ideally, the ligands coated on the surface of the larger microspheres react with the analyte first. After the reaction is complete, if there are still unreacted analytes (binding sites of analytes that have not been bound by the ligands), these unreacted analytes then react with the ligands coated on the surface of the smaller microspheres. In this way, the advantages of both ligands can be fully utilized, enabling detection regardless of whether the concentration of the analyte in the sample is low or high. However, due to the coexistence of both types of microspheres within the same reagent, ligands coated on the surfaces of different microspheres may simultaneously react with the analyte in the sample. For instance, at low concentrations, ligands on the smaller microspheres bind to some of the analyte, preventing full amplification of the reaction signal and thereby hindering the full exploitation of the advantages offered by both ligands.

Some improvement methods have been proposed to address the aforementioned issues. For example, ligands with different affinities to the analyte are used to coat microspheres of varying particle sizes. Ligands with higher affinity are coated on the surface of larger microspheres to facilitate the analyte binding first with the ligands on the surface of the larger microspheres. However, this method does not completely resolve the aforementioned issues. Another example is the proposal of a method where microspheres of two different sizes are prepared as two separate reagents. By sequentially adding these reagents, it is ensured that the ligands in the first reagent fully react with the analyte in the sample before reacting with the ligands in the second reagent, thereby completely avoiding the issue of both types of microspheres simultaneously binding with the analyte. However, the inventors have found that this method introduces a background signal when adding the second reagent, as the reaction system containing the first reagent and the sample is affected by changes in the total volume and alterations in the optical properties of the reaction solution caused by the microspheres in the second reagent. This results in inaccurate final detection results. Moreover, because microspheres of different sizes have different optimal main detection wavelengths, to obtain better detection performance, it is necessary to select a combination of microsphere size and concentration that is generally suitable for the same wavelength. As a result, the formulation of the reagent is subject to significant limitations, the expansion of linearity is not ideal, and certain detection requirements remain unmet.

Based on the above issues, the present disclosure proposes an improved sample analysis method, in which two reagents, each containing a ligand capable of binding to the analyte in the sample, are sequentially mixed and reacted with the sample. The signals obtained from the reaction of the analyte with the first reagent and the reaction with the second reagent are acquired, and the final detection result is determined based on the signals obtained from the reactions of the two stages. Firstly, unlike traditional methods that only utilize the signal at the reaction endpoint to obtain the detection result, the above-mentioned technical solution of the present disclosure fully utilizes the signal from the reaction of the first reagent with the analyte in the sample, as well as the signal from the further reaction after the addition of the second reagent, and uses the signals from the reactions of both stages to obtain the detection result. Secondly, in the above-described technical solution of the present disclosure, not only are the two reagents added sequentially, but the signals generated by the reactions of the two stages are also acquired separately. This allows separate consideration of the reaction systems of the two stages, enabling the separate use of more accurate and sensitive detection methods, thereby further expanding the lower and/or upper limits of detection. For example, different wavelengths (or combinations of primary and secondary wavelengths) of detection lights, or even different optical detection systems such as scattered light detection and transmitted light detection, can be used at different reaction stages. This allows more selectivity in the types of carriers, particle sizes, ligand affinities, etc., of the two reagents. For another example, the sample in the first-stage reaction system can be made relatively more concentrated to improve reaction sensitivity at low concentrations, and/or the sample in the second-stage reaction system can be made relatively more diluted to eliminate the hook effect at high concentrations or avoid exceeding the detection limit of the equipment, among other possibilities, but not limited to these. The following description of different embodiments further illustrates the technical solution of the present disclosure and its advantages in various aspects. Moreover, such a method is not limited to application in immunoturbidimetry, but can be extended to other detection methods such as non-immunologic turbidimetry or immunocolorimetry.

Therefore, the present disclosure provides a sample analysis method. The method is particularly an immunoturbidimetric assay.

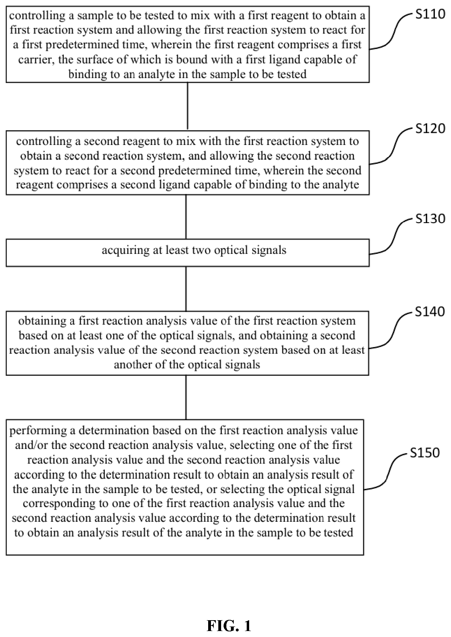

Referring to FIG. 1, according to one example, the sample analysis method comprises the following steps:
S110, controlling the sample to be tested to mix with a first reagent to obtain a first reaction system, and allowing the first reaction system to react for a first predetermined time, wherein the first reagent comprises a first carrier, the surface of which is bound with a first ligand capable of binding to an analyte in the sample to be tested;
S120, controlling a second reagent to mix with the first reaction system to obtain a second reaction system, and allowing the second reaction system to react for a second predetermined time, wherein the second reagent comprises a second ligand capable of binding to the analyte;
S130, acquiring at least two optical signals;
S140, obtaining a first reaction analysis value of the first reaction system based on at least one of the optical signals, and obtaining a second reaction analysis value of the second reaction system based on at least another of the optical signals;
S150, performing a determination based on the first reaction analysis value and/or the second reaction analysis value, and selecting one of the first reaction analysis value and the second reaction analysis value according to the determination result to obtain an analysis result of the analyte in the sample to be tested, or selecting the optical signal corresponding to one of the first reaction analysis value and the second reaction analysis value according to the determination result to obtain an analysis result of the analyte in the sample to be tested.

In the sample analysis method disclosed herein, the sample to be tested is sequentially treated with the first reagent and the second reagent, and at least two optical signals are acquired during the treatment process. Based on the acquired at least two optical signals, the analysis result of the analyte in the sample to be tested is finally obtained.

In S110, the sample to be tested is controlled to be mixed with the first reagent to obtain a first reaction system, and the first reaction system is allowed to react for a first predetermined time. The first reagent comprises a first ligand coated on the surface of a first carrier. The first ligand is capable of binding to the analyte in the sample to be tested. The first reaction system is obtained by mixing the first reagent with the sample to be tested, and the first reaction system is further allowed to react for a first predetermined time, for example, to be incubated at a certain temperature for a period of time. Generally, for an immune reaction (e.g., antibody-antigen reaction), incubation can be performed at approximately 37±3°C for several minutes (e.g., 1-5 minutes, preferably 3-5 minutes) to allow the first ligand to fully bind to the analyte. In S110, the analyte undergoes a first-stage reaction with the first ligand in the first reagent.

In the first reagent, the first ligand is coated on the surface of the first carrier. When the first ligand binds to the analyte in the first reaction system, the agglutination effect causes a significant change in the optical signal of the first reaction system. This allows the detection of the optical signal to reflect the concentration information of the analyte bound to the first ligand. When the concentration of the analyte in the sample to be tested is relatively low, all or most of the analyte will react with the first ligand. Typically, a carrier with larger particle size (or a carrier with significant spectral change after agglutination, such as colloidal gold) is used to increase the degree of change in the optical signal after the ligand reacts with the analyte, thereby improving detection sensitivity and broadening the detection lower limit.

Compared to the conventional method of adding a mixture of latex microspheres of two particle sizes in a single step, this method, by first mixing the first reagent with the sample to be tested and then allowing the first ligand to react with the analyte, avoids the possibility of ligands on both types of microspheres binding to the analyte after simultaneous mixing with the sample to be tested, which could lead to inaccurate results.

In S120, the second reagent is controlled to be mixed with the first reaction system to obtain a second reaction system, and the second reaction system is allowed to react for a second predetermined time. The second reagent comprises a second ligand capable of binding to the analyte.

The second ligand exists in the second reagent in a free form, or it may be coated on the surface of the second carrier. When unreacted analytes remain in the first reaction system, they bind to the second ligand in the second reagent after reacting for a second predetermined time, which causes a further significant change in the optical signal. In S120, unreacted analytes (if any) undergo a second-stage reaction with the second ligand in the second reagent.

The second ligand in the second reagent may exist in the second reagent in a form bound to the surface of the second carrier. Because the method allows the sample to be tested to react with the two reagents sequentially, and acquires the optical signals of the reactions separately, the two carriers can be chosen from a broader range and allow for more flexible combinations. The second carrier may be different from or identical to the first carrier. According to one example, a carrier with a smaller particle size relative to the first carrier is used as the second carrier. Because using a carrier with a smaller particle size results in a relatively smaller change in the optical signal after the ligand binds to the analyte, it is suitable for situations where the concentration of the analyte in the sample is high, thereby expanding the detection upper limit. In some detection examples, the second ligand can exist in a free form in the second reagent. However, the present disclosure is not limited to this. For example, the particle size of the first carrier, which will be detailed below, can be smaller than the particle size of the second carrier.

In the method, there are no particular limitations on the mixing time of the sample to be tested with the first reagent, or on the mixing time of the second reagent with the first reaction system. Those skilled in the art can determine this according to actual needs. The exemplary mixing time is typically less than 1 minute, such as 10s, 20s, 30s, 40s, 50s, etc. During the mixing stage, it is understood that the reagent is not yet uniformly dispersed, and the ligand in the reagent has not yet fully come into contact with the analyte; therefore, essentially no reaction or only a minimal amount of reaction has occurred.

After mixing, the first and second reaction systems are further allowed to react for a predetermined time. In the method, there are no particular limitations on the length of the first predetermined time and the second predetermined time. Those skilled in the art can determine the time based on actual needs, such as the specific analyte and the first and second ligands used. For example, the first and second predetermined times are greater than 1 minute, such as 1-5 minutes, preferably 3-5 minutes, specifically, such as 3 minutes, 4 minutes, or 5 minutes.

The term "incubation" as used herein generally refers to holding the mixture of the reagent and sample at a certain temperature for a period of time to allow sufficient reaction of the reactants. The incubation time is typically calculated from the moment the reactants are added together in the detection device. Therefore, the "incubation" process comprises both the procedure of mixing and the procedure of reaction for (first or second) predetermined time as described above.

In S130, at least two optical signals are acquired.

In S140, the first reaction analysis value for the first reaction system is obtained based on at least one of the optical signals, and the second reaction analysis value for the second reaction system is obtained based on at least another of the optical signals.

In the method described, the at least two optical signals can respectively reflect the binding of the analyte to the first ligand and the further binding of the analyte to the second ligand, and accordingly, the first reaction analysis value for the first reaction system is obtained based on at least one of the optical signals, and the second reaction analysis value for the second reaction system is obtained based on at least another of the optical signals.

In some embodiments of the present disclosure, obtaining the reaction analysis value based on the optical signal may be achieved using only one optical signal. In some cases, the optical signal itself can be directly used as the reaction analysis value. In other cases, after processing (e.g., correction, multiplication by a coefficient (e.g., volume correction coefficient, weight coefficient, etc.), or subtraction of instrument background signal, etc.), this optical signal is used as the reaction analysis value.

Furthermore, in other embodiments, obtaining the reaction analysis value based on the optical signal may also be achieved by calculating the reaction analysis value using two or more optical signals. For example, the optical signal of the reagent or the optical signal immediately after the reagent is mixed with the sample is first measured as the blank optical signal. Then, the reaction analysis value is obtained by subtracting this blank optical signal from the optical signal at the reaction endpoint of the reaction system. For another example, multiple optical signals (e.g., reaction kinetic curves) may be obtained throughout or during part of the reaction process, and the reaction analysis value may be derived from all or a portion of these multiple optical signals. Similarly, one, several, or all of the two or more optical signals may also undergo processing before being used to calculate the reaction analysis value. In still other embodiments, the reaction analysis value may further be a preliminary analysis result (e.g., concentration) of the analyte obtained based on a standard curve (also referred to as a calibration curve). For instance, a correspondence (i.e., standard curve) between optical signals and analyte concentration values may be established in advance. One or more collected optical signals can then be matched with the standard curve to obtain the corresponding analyte concentration value, which serves as the reaction analysis value.

Unlike conventional methods that only detect the optical signal at the endpoint of the reaction after all analytes have bound to the ligand, the method disclosed herein acquires at least two optical signals in S130. These signals are obtained from steps S110 and S120, respectively reflecting at least two optical signals indicative of the first-stage reaction and the second-stage reaction. Furthermore, the first reaction analysis value of the first reaction system is derived based on at least one of the optical signals, and the second reaction analysis value of the second reaction system is derived based on at least another of the optical signals.

Further, in S150, the determination is performed based on the first reaction analysis value and/or the second reaction analysis value, the analysis result of the analyte in the sample to be tested is obtained by selecting one of the first reaction analysis value and the second reaction analysis value according to the determination result; or, the analysis result of the analyte in the sample to be tested is obtained by selecting the optical signal corresponding to one of the first reaction analysis value and the second reaction analysis value according to the determination result.

Due to the wide fluctuation in the concentration of the analyte in the sample to be tested, for different samples, there are situations where the detection results from the first-stage reaction better reflect the analyte concentration, as well as situations where the detection results from the second-stage reaction better reflect the analyte concentration. The method involves performing the determination based on the first reaction analysis value and/or the second reaction analysis value, selecting one of them based on the determination result, and further obtaining the analysis result of the analyte in the sample to be tested. Compared to conventional methods, this method achieves improved detection accuracy over a wider range of analyte concentrations, thereby significantly extending the linear detection range of the method.

As mentioned above, the reaction analysis value can be a single optical signal itself, a processed optical signal, or a reaction analysis value calculated from multiple optical signals. Therefore, in the described method, after selecting one of the first reaction analysis value and the second reaction analysis value, according to one example, the analysis result can be obtained through the selected reaction analysis value. Alternatively, according to another example, even if the reaction analysis value is not the optical signal itself, the unprocessed optical signal corresponding to that reaction analysis value is still used to obtain the analysis result.

The method for obtaining the analysis result of the analyte in the sample to be tested through the reaction analysis value or its corresponding optical signal can be any conventional method. For example, a standard curve (or calibration curve) can be obtained in advance by detecting a series of standard samples containing known concentrations of the analyte. Corresponding to the method disclosed herein, a first standard curve measured from the reaction of standard samples with the first reagent and a second standard curve measured from the reaction of standard samples with the first reagent and further with the second reagent can be obtained separately. After selecting one of the first reaction analysis value and the second reaction analysis value of the sample to be tested using the method described herein, the concentration of the analyte in the sample to be tested can be obtained based on the corresponding standard curve using the selected reaction analysis value or its corresponding optical signal. Alternatively, when the reaction analysis value itself serves as a preliminary analysis result (e.g., concentration) of the analyte, this reaction analysis value can be directly used/output as the final analysis result of the analyte.

The method disclosed herein can obtain more accurate analysis results and significantly broaden the detection linearity range of the method by utilizing the first reaction analysis value and the second reaction analysis value that respectively reflect the reactions of the two-stages.

In some embodiments, S150, performing a determination based on the first reaction analysis value and/or the second reaction analysis value and further obtaining an analysis result of the analyte in the sample to be tested, comprises: comparing the first reaction analysis value with the second reaction analysis value, and based on the comparison result, selecting one of the first reaction analysis value and the second reaction analysis value to obtain the analysis result of the analyte in the sample to be tested; or selecting the optical signal corresponding to one of the first reaction analysis value and the second reaction analysis value based on the comparison result to obtain the analysis result of the analyte in the sample to be tested.

In some embodiments, the first reaction analysis value and the second reaction analysis value are compared. Based on the comparison result, the larger one of the first reaction analysis value and the second reaction analysis value is selected to obtain the analysis result of the analyte in the sample to be tested. Alternatively, the optical signal corresponding to the larger one of the first reaction analysis value and the second reaction analysis value is selected based on the comparison result to obtain the analysis result of the analyte in the sample to be tested.

In some other embodiments, it is determined whether the first reaction analysis value or the second reaction analysis value falls within a preset range. According to the determination result, one of the first reaction analysis value and the second reaction analysis value is selected to obtain the analysis result of the analyte in the sample to be tested or according to the determination result, the light signal corresponding to one of the first reaction analysis value and the second reaction analysis value is selected to obtain the analysis result of the analyte in the sample to be tested.

In the various embodiments described, the one that more accurately reflects the concentration of the analyte in the sample to be tested is selected by performing a determination based on the first reaction analysis value and the second reaction analysis value. As mentioned above, the concentration of the analyte in the sample to be tested may vary within a wide range. Therefore, when the concentration of the analyte is low, the analyte essentially binds completely to the first ligand in the first reagent. After the second reagent is added, changes in the optical signal are primarily due to the components of the second reagent and the change in system volume. In contrast, when the analyte concentration is high, a significant amount of unreacted analyte remains after binding with the first ligand in the first reagent. Upon addition of the second reagent, these unreacted analytes further bind to the second ligand, causing the optical signal to continuously increase. Thus the one that more accurately reflects the analyte concentration can be selected according to the determination based on the first reaction analysis value and the second reaction analysis value.

One method of determination involves comparing the first reaction analysis value with the second reaction analysis value. In a preferred embodiment, the larger of the two values is selected, and the analysis result of the analyte in the sample to be tested is further obtained based on this value. Another method of determination comprises, for example, comparing the first reaction analysis value with a first preset range. If the first reaction analysis value falls within the first preset range (or alternatively, it may be set to fall outside the first preset range), the concentration of the analyte in the sample to be tested is further derived from this first reaction analysis value. If the first reaction analysis value falls outside the first preset range (or alternatively, it may be set to fall within the first preset range), the second reaction analysis value is selected, and the concentration of the analyte in the sample to be tested is further derived from this second reaction analysis value. In this determination method, the second reaction analysis value may also be compared with a second preset range. In other embodiments, the first reaction analysis value and the second reaction analysis value may be compared with their respective preset ranges. Based on the comparison results, an appropriate reaction analysis value is selected to obtain the analysis result of the analyte. The preset range may also be a threshold value. The preset range can be determined empirically. Typically, the preset range is related to specific equipment, the detection method set by the equipment, the detection item (i.e., sample type and specific analyte), and the reagents used. Those skilled in the art can determine an appropriate preset range based on actual circumstances.

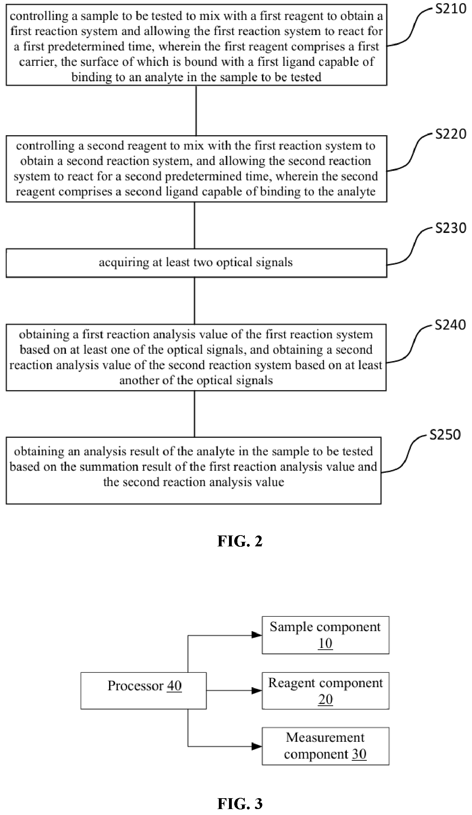

Referring further to FIG. 2, a sample analysis method according to another example is shown. The method comprises the following steps:
S210, controlling a sample to be tested to mix with a first reagent to obtain a first reaction system, and allowing the first reaction system to react for a first predetermined time, wherein the first reagent comprises a first carrier, the surface of which is bound with a first ligand capable of binding to an analyte in the sample to be tested;
S220: controlling a second reagent to mix with the first reaction system to obtain a second reaction system, and allowing the second reaction system to react for a second predetermined time, wherein the second reagent comprises a second ligand capable of binding to the analyte;
S230: acquiring at least two optical signals;
S240: obtaining a first reaction analysis value of the first reaction system based on at least one of the optical signals, and obtaining a second reaction analysis value of the second reaction system based on at least another of the optical signals;
S250: obtaining an analysis result of the analyte in the sample to be tested based on the summation result of the first reaction analysis value and the second reaction analysis value.

In this embodiment, S210 to S240 are the same as S110 to S140 in the foregoing embodiments, and will not be described again herein

Unlike the foregoing embodiments where the final result was obtained by selecting one of the first reaction analysis value and the second reaction analysis value according to a determination method, in S250, the final analysis result is obtained based on the summation result of the first reaction analysis value and the second reaction analysis value.

Similarly, the at least two optical signals can be directly used to obtain the sum of the first reaction analysis value and the second reaction analysis value, or through processing, and optionally by further assigning different weights to obtain the sum of the first reaction analysis value and the second reaction analysis value.

In this embodiment, the sum of the first reaction analysis value and the second reaction analysis value, which reflect the reactions of the two stages, is utilized, thereby obtaining a more accurate analysis result and significantly broadening the detection linearity range of the method. This approach, first, reflects the reactions of the two stages in the final result, thus broadening the detection range, and second only requires one standard curve. It is more suitable for situations where the first reaction does not exhibit a severe hook effect. Furthermore, to obtain better detection performance, the first reaction analysis value and the second reaction analysis value can be weighted before summing.

In some embodiments, different methods can be used to obtain the analysis result of the analyte in the sample to be tested based on the summation result. One example is to obtain the analyte concentration from the summation result using a corresponding standard curve. Another example is to perform further calculations on the summation result such as averaging, weighted averaging, or other operations, to obtain the analysis result of the analyte in the sample to be tested.

In other embodiments, for example, based on relevant information obtained during the reaction process-such as interference conditions, antigen excess, or information known prior to the reaction, such as the sample concentration range, the presence of interferents or analogs in the sample-the more reliable reaction analysis value is selected to obtain the analysis result of the analyte in the sample to be tested.

In some embodiments of the above two examples, acquiring at least two optical signals in S130 and S230 comprises the following steps:
acquiring at least one optical signal after controlling the sample to be tested to mix with the first reagent and before controlling the second reagent to mix with the first reaction system, and acquiring at least another optical signal after controlling the second reagent to mix with the first reaction system;
alternatively, acquiring the at least two optical signals after controlling the second reagent to mix with the first reaction system.

In the method, the at least two optical signals are further used to obtain a first reaction analysis value for the first reaction system and a second reaction analysis value for the second reaction system, respectively. Therefore, according to some embodiments, at least one of the optical signals is acquired after the first reagent and the sample to be tested are mixed but before the addition of the second reagent, and at least another of the optical signals is acquired after the addition of the second reagent. Thus, the at least two optical signals are acquired during the first-stage reaction and the second-stage reaction, respectively. According to other embodiments, the at least two optical signals are all acquired after the addition of the second reagent. In such cases, immediately after the second reagent is added to the first reaction system (e.g., within 1 minute of addition, for example at 10 s, 20 s, 30 s, or 40 s), the second ligand in the second reagent has not yet participated significantly in the reaction. At this point, compared to the optical signal of the system after the sample to be tested and the first reagent have reacted for the first predetermined time (i.e., at the endpoint of the first reaction stage), the change in the system's optical signal primarily originates from components in the second reagent (such as the second carrier) and the change in optical signal caused by the increased volume of the system. In some instances, this change in the system's optical signal is sufficiently small or stable enough not to introduce significant noise. The at least one optical signal acquired at this stage primarily reflects the change in the system's optical signal resulting from the first-stage reaction; therefore, the first reaction analysis value of the first reaction system can be derived from it. During or after the second predetermined time, at least another optical signal is further acquired, which then additionally incorporates the change in the system's optical signal caused by the second-stage reaction; thus, the second reaction analysis value of the second reaction system can be obtained therefrom.

In some embodiments of the above two examples, acquiring at least two optical signals in S130 and S230 comprises acquiring the at least one of the optical signals at a first detection wavelength and acquiring the at least another optical signal at a second detection wavelength.

In the method of the present disclosure, the first ligand and the second ligand in the first reagent and the second reagent are respectively bound to the carrier surface, or the second ligand may exist in a free form. Therefore, the optimal detection wavelength for the first reaction system after the addition of the first reagent may or may not be suitable for the second reaction system after the further addition of the second reagent. In other words, the optimal detection wavelengths for the first reaction system and the second reaction system may be different.

Therefore, preferably, the first detection wavelength and the second detection wavelength are different. By setting optimal detection wavelengths suitable for the first and second reaction systems respectively, the sensitivity and accuracy of detection can be further improved, and the linear range of detection can also be further extended. Generally, shorter wavelengths are beneficial for improving detection sensitivity, while longer wavelengths are beneficial for detecting high-concentration samples, preventing excessively strong optical signals that could exceed the detection limit of the device.

In traditional detection methods, a fixed wavelength is often used for detecting the same sample. Therefore, when the reagent contains two kinds of microspheres with different particle sizes, whether they are mixed in one reagent or, as in the method disclosed herein, separately contained in two reagents, the particle sizes of the two kinds of microspheres selected must be approximately suitable for the detection wavelength. This imposes certain limitations on the selection of microspheres. However, in the above embodiments, since different detection wavelengths can be used during the detection of the same sample, the range of carriers that can be selected for the first reagent and the second reagent can be expanded. Furthermore, by selecting carriers with larger differences in particle size, the lower and upper limits of detection can be further broadened.

In some specific embodiments, the first detection wavelength and the second detection wavelength are selected from 320 nm to 860 nm; preferably, the first detection wavelength and the second detection wavelength are selected from the range of 340 nm to 850 nm.

In some embodiments, the detection wavelength consists of a primary detection wavelength and a secondary detection wavelength.

The following further describes several exemplary embodiments of acquiring, processing, and analyzing optical signals in the two examples described above. It should be understood that the sample detection method is not limited to these exemplary embodiments.

According to a first embodiment, acquiring at least two optical signals in S130 and S230 respectively comprises the following steps:
acquiring a first optical signal based on the first reagent, or acquiring a first optical signal after controlling the sample to be tested to mix with the first reagent and before the start of the first predetermined time;
acquiring a second optical signal after the end of the first predetermined time and before controlling the second reagent to mix with the first reaction system;
acquiring a third optical signal after controlling the second reagent to mix with the first reaction system and before the start of the second predetermined time;
acquiring a fourth optical signal after the end of the second predetermined time.

In S140 and S240, "obtaining a first reaction analysis value of the first reaction system based on at least one of the optical signals and obtaining a second reaction analysis value of the second reaction system based on at least another of the optical signals respectively" comprises: obtaining a first reaction analysis value of the first reaction system based on the first optical signal and the second optical signal, and obtaining a second reaction analysis value of the second reaction system based on the third optical signal and the fourth optical signal.

In this embodiment, the second and fourth optical signals are acquired at the endpoint of the first-stage reaction and the endpoint of the second-stage reaction, respectively. Thus, the second and fourth optical signals respectively comprise the concentration information of the analyte participating in the first-stage reaction and the concentration information of the analyte participating in both the first-stage reaction and the second-stage reaction (i.e., the total concentration information of the analyte). Further, a first optical signal is acquired immediately upon the addition of the first reagent or immediately after mixing the first reagent with the sample to be tested, and a third optical signal is acquired immediately after mixing the second reagent with the first reaction system. Thus, the first optical signal comprises the optical signal of the first reagent itself or the optical signal when the first reagent and the sample to be tested have just been mixed before the analyte and the first ligand have reacted significantly. This first optical signal can be considered as a blank signal of the first reaction system. Similarly, the third optical signal can be considered as a blank signal of the second reaction system.

Obtaining the first reaction analysis value of the first reaction system based on the first optical signal and the second optical signal may involve, for example, subtracting the first optical signal from the second optical signal, or processing (e.g., correcting) the second optical signal and/or the first optical signal and then subtracting the processed first optical signal from the processed second optical signal, thereby obtaining the first reaction analysis value.

Similarly, obtaining the second reaction analysis value of the second reaction system based on the third optical signal and the fourth optical signal may involve, for example, subtracting the second optical signal from the fourth optical signal, or processing (e.g., correcting) the fourth optical signal and/or the second optical signal and then subtracting the processed second optical signal from the processed fourth optical signal, thereby obtaining the second reaction analysis value.

In the method, "after controlling the sample to be tested to mix with the first reagent and before the start of the first predetermined time" and "after controlling the second reagent to mix with the first reaction system and before the start of the second predetermined time" respectively refer to a specific time point (e.g., within approximately 1 minute, 50 seconds, or 40 seconds) after adding the first reagent to the sample to be tested or the second reagent to the first reaction system. Examples of such time points are 10 seconds, 20 seconds, 30 seconds, 40 seconds, or 50 seconds.

According to a second embodiment, acquiring at least two optical signals in S130 and S230 respectively comprises the following steps:
acquiring a second optical signal after the end of the first predetermined time and before controlling the second reagent to mix with the first reaction system;
acquiring a fourth optical signal after the end of the second predetermined time.

In S140 and S240, "obtaining a first reaction analysis value of the first reaction system based on at least one of the optical signals and obtaining a second reaction analysis value of the second reaction system based on at least another of the optical signals respectively" comprises: obtaining a first reaction analysis value of the first reaction system based on the second optical signal, and obtaining a second reaction analysis value of the second reaction system based on the fourth optical signal or based both on the fourth optical signal and the second optical signal.

In this embodiment, the second and fourth optical signals are acquired at the endpoints of the first-stage reaction and the second-stage reaction, respectively. The second optical signal and the fourth optical signal can be directly used as the first reaction analysis value and the second reaction analysis value, respectively, or the second optical signal and the fourth optical signal can be processed as described above, and then used as the first reaction analysis value and the second reaction analysis value, respectively. Furthermore, the second optical signal can be used as a blank signal for the second-stage reaction, and the second optical signal can be subtracted from the fourth optical signal.

As mentioned above, after adding the second reagent, the system's optical signal undergoes changes unrelated to the analyte. This is caused by the inconsistency in absorbance between the second reagent and the reaction system before the addition of the second reagent, a phenomenon observable when testing zero-concentration samples. As a result, the optical signal measured during the second-stage reaction comprises not only the reaction between the reagent and the analyte but also this background signal unrelated to the analyte. This phenomenon reduces the signal-to-noise ratio. That is, the change in absorbance due to the reaction between the analyte and the reagent may be overshadowed by the larger change in absorbance caused by the reaction system itself. Therefore, in conventional methods, it is necessary to make a balance in blank absorbance between the first and second reagents. To minimize the difference between them, the choice of carriers and carrier particle sizes is limited.

This phenomenon can be addressed by collecting the optical signal after the addition of the second reagent as a blank optical signal (as in the method of the first embodiment).

When the second optical signal is used as the blank signal for the second-stage reaction, the second optical signal cannot reflect the absorbance change caused by dilution after the addition of the second reagent. The influence of this phenomenon can be eliminated by multiplying the second optical signal by a volume correction factor. The volume correction $factor = \left(\frac{{V}_{R 1} + {V}_{S}}{{V}_{R 1} + {V}_{S} + {V}_{R 2}}\right)$, that is, the ratio of the sum of the first reagent volume V_{R1} and the sample volume V_{S} to the sum of the first reagent volume V_{R1}, the second reagent volume V_{R2}, and the sample volume V_{S}.

According to a third embodiment, acquiring at least two optical signals in S130 and S230 respectively comprises the following steps:
acquiring a third optical signal after controlling the second reagent to mix with the first reaction system and before the start of the second predetermined time;
acquiring a fourth optical signal after the end of the second predetermined time.

In S140 and S240, "obtaining a first reaction analysis value of the first reaction system based on at least one of the optical signals, and obtaining a second reaction analysis value of the second reaction system based on at least another of the optical signals" respectively comprises: obtaining a first reaction analysis value of the first reaction system based on the third optical signal, and obtaining a second reaction analysis value of the second reaction system based on the fourth optical signal or based both on the fourth optical signal and the third optical signal.

In this embodiment, the at least two optical signals are obtained after the addition of the second reagent. As previously described, immediately after the second reagent is added to the first reaction system (e.g., within 1 minute of addition, for example at 10 s, 20 s, 30 s, or 40 s), the second ligand in the second reagent has not yet participated significantly in the reaction. At this time, the at least one optical signal obtained primarily reflects changes in the optical signal of the system due to the first-stage reaction. Therefore, the first reaction analysis value of the first reaction system can be derived from this. During or after the second predetermined period, further obtaining at least another optical signal additionally captures changes in the system's optical signal resulting from the second-stage reaction. Consequently, the second reaction analysis value of the second reaction system can be determined from this. Specifically, the second reaction analysis value of the second reaction system can be obtained solely based on the fourth optical signal; alternatively, the second reaction analysis value of the second reaction system can be obtained based on both the fourth optical signal and the third optical signal (for example, using the third optical signal as the blank signal for the second-stage reaction, and subtracting the third optical signal from the fourth optical signal).

According to a fourth embodiment, acquiring at least two optical signals in S130 and S230 respectively comprises the following steps:
acquiring a first optical signal based on the first reagent or acquiring a first optical signal after controlling the sample to be tested to mix with the first reagent and before the start of the first predetermined time;
acquiring a second optical signal after the end of the first predetermined time and before controlling the second reagent to mix with the first reaction system;
acquiring a fourth optical signal after the end of the second predetermined time.

In S140 and S240, "obtaining the first reaction analysis value of the first reaction system based on at least one of the optical signals, and obtaining the second reaction analysis value of the second reaction system based on at least another of the optical signals" respectively comprises: obtaining the first reaction analysis value of the first reaction system based on the first optical signal and the second optical signal, and obtaining the second reaction analysis value of the second reaction system based on the first optical signal and the fourth optical signal, or based on the second optical signal and the fourth optical signal.

This embodiment differs from the aforementioned second embodiment in that it further obtains the first optical signal immediately after the first reagent is added, or immediately after the first reagent is mixed with the sample to be tested. As mentioned above, the particle size of the first carrier in the first reagent can be relatively large, thereby increasing the intensity of the optical signal for the same sample to be tested relative to a reagent containing a carrier with smaller particle size, thus improving detection sensitivity and broadening the detection lower limit. Therefore, the optical signal of the first reagent itself is also stronger than that of the second reagent containing a carrier with smaller particle size or even not containing a carrier. By subtracting the optical signal of the first reagent itself or the optical signal after the first reagent is mixed with the sample to be tested but before the start of the first predetermined time from the optical signal at the reaction endpoint (i.e., the second optical signal), the accuracy of the first reaction analysis value of the first reaction system can be improved. In one example, the first optical signal can be subtracted from the fourth optical signal to obtain the second reaction analysis value of the second reaction system, which also improves the accuracy of the second reaction analysis value.

According to a fifth embodiment, obtaining at least two optical signals in S130 and S230 respectively comprises the following steps:
collecting multiple optical signals during at least a period from the start of the first predetermined time to the end of the first predetermined time, and obtaining a first reaction curve;
collecting multiple optical signals during at least a period from the start of the second predetermined time to the end of the second predetermined time, thereby obtaining a second reaction curve.

In S140 and S240, "obtaining the first reaction analysis value of the first reaction system based on at least one of the optical signals, and obtaining the second reaction analysis value of the second reaction system based on at least another of the optical signals" respectively comprises: obtaining the first reaction analysis value of the first reaction system based on the first reaction curve, and obtaining the first reaction analysis value of the first reaction system based on the second reaction curve.

In this embodiment, unlike the sampling method in the first to fourth embodiments described above, a rate method is used to obtain the reaction analysis values. Generally, when the reactant concentration in the reaction system is high, the reaction rate is fast; conversely, when the reactant concentration is low, the reaction rate is slow. Therefore, this embodiment utilizes the change in reaction rate (i.e., reaction kinetic information) reflected by the change in the optical signal to obtain the concentration information of the analyte in the sample to be tested.

By collecting multiple optical signals over at least a period from the start of the first predetermined time to the end of the first predetermined time, at least a portion of the reaction curve of the first-stage reaction (first reaction curve) can be obtained, thereby obtaining the reaction kinetic information of the first-stage reaction. Similarly, at least a portion of the reaction curve of the second-stage reaction (second reaction curve) can be obtained, thereby obtaining the reaction kinetic information of the second-stage reaction.

In a preferred embodiment, the first reaction analysis value of the first reaction system is obtained based on the first reaction curve or based on a characteristic value of the first reaction curve, and the second reaction analysis value of the second reaction system is obtained based on the second reaction curve or based on a characteristic value of the second reaction curve.

Among which, collecting multiple optical signals comprises, for example, collecting multiple optical signals at fixed time intervals within a certain time period. For example, during the first predetermined time or the second predetermined time, or during "incubation", optical signals are continuously collected at fixed time intervals, such as 10s or 20s, over a duration of, for example, 1 to 5 minutes (e.g., 2 minutes, 3 minutes, 4 minutes).

The characteristic value, for example, may include the slope of a certain curve (the rate of change of the optical signal per unit time), the maximum slope (the extreme value of the rate of change of the optical signal per unit time), the integral of the slope over time, and so on, but are not limited to these.

The above describes five preferred embodiments of the sample analysis method, in which at least two optical signals are acquired, a first reaction analysis value of the first reaction system and a second reaction analysis value of the second reaction system are further obtained based on the at least two optical signals, and the analysis result of the analyte in the sample to be tested is further obtained based on the first reaction analysis value and the second reaction analysis value via a determination-based method or a summation-based method.

It should be understood that the sample analysis method of the present disclosure is not limited to the five embodiments described above.

In some embodiments, for example, when detecting the two stage reactions using different wavelengths, a second optical signal can be acquired at a first detection wavelength after the first predetermined time and before the addition of the second reagent, and a third optical signal can be acquired at a second detection wavelength different from the first detection wavelength. The second optical signal serves as the optical signal at the endpoint of the first-stage reaction, and the third optical signal serves as a blank signal for the second-stage reaction. In other embodiments, similarly, after adding and mixing the second reagent and before the start of the second predetermined time, a second optical signal can also be acquired using a first detection wavelength, and a third optical signal can be acquired using a second detection wavelength different from the first detection wavelength. The second optical signal serves as the optical signal at the endpoint of the first-stage reaction, and the third optical signal serves as a blank signal for the second-stage reaction.

For further example, based on the various embodiments, the ratio of the sample to be tested to the reagent added can be adjusted to further adjust the linear range applicable to the detection method, and improve the accuracy of the detection. According to one example, a first reagent with a reduced volume compared to conventional practices can be mixed with a conventional volume of the sample to be tested to relatively increase the concentration of the analyte in the obtained first reaction system, thereby further improving the detection sensitivity of the first reagent. In this example, by controlling the amount of reagent and sample added, the concentration of the analyte in the first reaction system increases relatively. According to another example, a sample to be tested with a reduced volume compared to conventional practices can be mixed with a first reagent with a proportionally reduced volume. At this time, the concentrations of various components in the first reaction system remain unchanged compared to conventional practices, only the system volume is reduced. Then, a second reagent with an increased volume is added to relatively reduce the concentration of the analyte in the obtained second reaction system thereby further increasing the detection upper limit. In this example, by controlling the amounts of reagent and sample added, the concentration of the analyte in the second reaction system is relatively diluted. Here, to maintain low reagent costs, the concentration of the ligand (and/or carrier) of the second reagent can be reduced while increasing the volume of the second reagent. Other adjustments to volume and/or concentration can also be used to improve the detection sensitivity and/or linear detection upper limit.

According to some embodiments, the binding affinity of the first ligand to the analyte is higher than the binding affinity of the second ligand to the analyte. Thus, when the first reagent is mixed with the sample to be tested, the first ligand binds more easily to the analyte, further improving the sensitivity of the detection method and thus further widening the lower limit of the detection method. Furthermore, by setting the binding affinity of the first ligand to the analyte to be higher than the binding affinity of the second ligand to the analyte, the detection sensitivity of the first reagent for samples containing low concentrations of the analyte is improved. This also allows for greater flexibility in the selection of the particle sizes of the first and (if present) second carriers. For example, a relatively small particle size of the first carrier can be selected, or even a particle size of the first carrier smaller than that of the second carrier can be selected.

In the method of the present disclosure, the second ligand in the second reagent exists in a free form, or in a form bound to the surface of the second carrier, or the second reagent may simultaneously comprise both the second ligand in free form and the second ligand in a form bound to the carrier surface.

In a preferred embodiment, the carrier is latex microspheres. However, the carrier used in the sample detection method is not limited to this. For example, the carrier can also be magnetic beads, colloidal gold, silver nanoparticles, polymerized proteins, etc. In summary, any substance that can form an aggregated state and induce a change in optical signal within the reaction system can serve as a carrier. Those skilled in the art can make selections based on actual needs.

In some embodiments, the first carrier in the first reagent comprises latex microspheres with a first particle size, and the second carrier in the second reagent comprises latex microspheres with a second particle size. The present disclosure does not impose any particular limitation on the particle size selection of the first carrier and the second carrier; for example, they can be the same, or the particle size of the first carrier can be different from that of the second carrier. In a preferred embodiment, the second particle size is smaller than the first particle size. As previously described, treating the sample to be tested with such first reaction and second reagent can achieve a lower linear detection lower limit and a higher linear detection upper limit.

The above embodiments are described with the assumption that each reagent contains only microspheres of one particle size. It should be understood that the method of the present disclosure is not limited thereto. For example, each reagent may comprise microspheres of two or more particle sizes, as long as combinations of particles with appropriate sizes and ligands are selected, making the first reagent suitable for detecting samples with low concentrations of the analyte to meet the requirement of a lower limit of linear detection, while making the second reagent suitable for detecting samples with high concentrations of the analyte to meet the requirement of an upper limit of linear detection.

In some embodiments, the method may further comprise further processing the sample to be tested with a third reagent.

According to one example, the third reagent is a buffer, and optionally may comprise an anti-interference agent. In this example, the third reagent is mixed with the sample to be tested before the addition of the first reagent, or the third reagent can be added after the first reagent has been added and reacted for the first predetermined time. The addition of the third reagent can dilute the sample to be tested to some extent. Furthermore, if the third reagent contains an anti-interference agent, by mixing the third reagent with the sample to be tested, interference can be eliminated, thereby improving the detection accuracy.

According to another example, the third reagent is a reagent with higher sensitivity. In this example, the third reagent is mixed with the sample to be tested before the addition of the first reagent, and reacted for a certain time, during which an optical signal is acquired, and a reaction analysis value is obtained based on this optical signal. Then, the first reagent and the second reagent are further added to further react with the analyte in the sample to be tested, and optical signals and corresponding reaction analysis values are acquired respectively. In this example, reaction analysis values for three stage reactions can be obtained, and the most suitable one can be selected from them using a method similar to those described above to obtain the final analysis result, or the three reaction analysis values can undergo calculation, and the final analysis result can be obtained based on the calculation result. The analysis method of this sample can further broaden the detection lower limit.

According to yet another example, the third reagent is suitable for samples to be tested containing high concentrations of the analyte, and used to broaden the detection upper limit. Similar to the previous examples, this third reagent is added to the reaction system after the reaction of the second reagent to further carry out the reaction, finally obtaining the reaction analysis values of the three stage reactions, and thus obtaining the final analysis result.

Furthermore, the methods of the above examples can also be combined to use four or even more reagents for detection.

According to some embodiments, the first ligand and the second ligand are antigens or antibodies capable of binding to the analyte.

The present disclosure does not particularly limit the type of ligand. In addition to antigens or antibodies, the sample detection method can also use other substances that have a certain binding ability to the analyte and whose reaction products can change the optical signal of the system and thus be detected, and examples thereof include aptamers, enzymes, or substrates. Those skilled in the art can make selection according to actual needs.

According to some embodiments, the optical signal comprises a scattered optical signal and/or a transmitted optical signal.

According to some embodiments, the sample to be tested is a bodily fluid sample from mammals, especially primates, particularly humans, such as blood, urine, cerebrospinal fluid, and saliva.

The present disclosure does not impose any particular limitation on the specific analytical items to which the sample analysis method described herein is applied. Any detection item that utilizes ligands for detection, such as immunoturbidimetry, can have its linear range broadened using the method of the present disclosure. Examples comprise immunoglobulins (IgA, IgG, IgE), complement components (C3, C1q), apolipoproteins (ApoA1, ApoE), haptens (e.g., antibiotics, vitamin D, digoxin), antibodies (e.g., anti-streptolysin O antibody (ASO), anti-cyclic citrullinated peptide antibody (ACCP), anti-*Helicobacter pylori* antibody), tumor markers (e.g., alpha-fetoprotein (AFP), carcinoembryonic antigen (CEA)), enzymes (e.g., creatine kinase isoenzyme (CKMB), lipoprotein-associated phospholipase A2 (LP-PLA2)), acute phase proteins (e.g., C-reactive protein, ceruloplasmin, haptoglobin), myocardial injury markers (myoglobin, troponin), and common proteins such as albumin, prealbumin, hemoglobin, glycated hemoglobin, cystatin C in body fluids. The method described herein is particularly suitable for detection items with large variations in analyte concentration, such as CRP, MALB, RF, β2-MG, etc., but is not limited to these.

The sample analysis method disclosed herein is not limited to enhanced immunoassay methods using latex microspheres. The method can utilize other carriers capable of enhancing the detection signal, such as colloidal gold immunocolorimetry. For example, suitable carrier materials comprise any inorganic, organic, or polymeric materials used in particle-enhanced light scattering assays. Such materials comprise, for example: selenium, carbon, gold; nitrides of carbon, silicon, or germanium, such as Si₃N₄; oxides of iron, titanium, or silicon, such as TiO₂, SiO₂; and polymeric materials such as polystyrene, polyvinyl chloride, epoxy resin, polyvinylidene chloride, poly(α-naphthalene methacrylate), polynaphthalene ethylene, or copolymers thereof, particularly copolymers of styrene and olefinic unsaturated compounds, such as styrene-(meth)acrylate copolymers, core-shell particles composed of a polystyrene core and a styrene-olefinic unsaturated compound copolymer shell, etc.

A second aspect of the present disclosure also provides a sample analyzer.

Before describing the sample analyzer in detail, its structure will be described first.

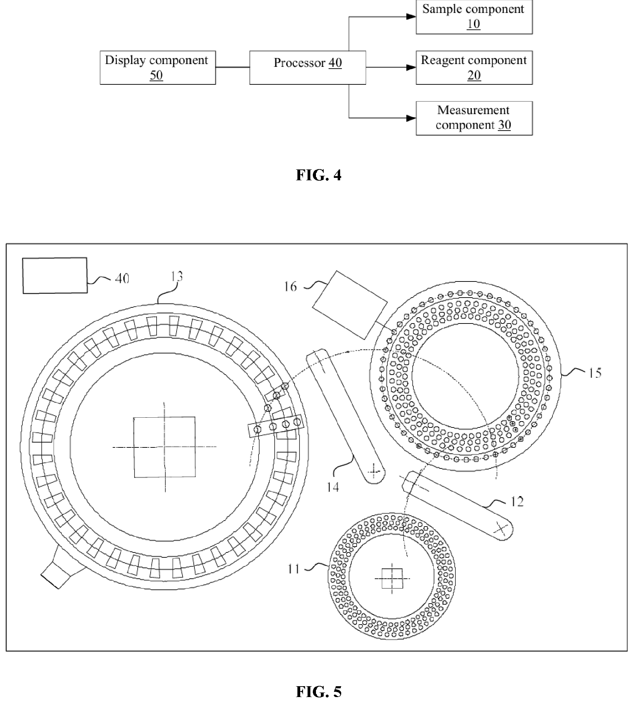

Referring to FIG. 3, one example of the sample analyzer is shown. In this example, the sample analyzer comprises a sample component 10, a reagent component 20, a measurement component 30, and a processor 40. According to another example, referring to FIG. 4, the sample analyzer also comprises a display component 50.

The sample component 10 is used to hold the sample to be tested, draw the sample and dispense it to the measurement component 30. Referring to FIG. 5, in some embodiments, the sample component 10 comprises a sample-carrying component 11 and a sample-dispensing mechanism 12. The sample-carrying component 11 is used to hold the sample. In some examples, the sample-carrying component 11 may comprise a sample delivery module (SDM) and a front-end track. In other examples, such as FIG. 5, the sample-carrying component 11 may also be a sample tray, and the sample tray comprises multiple sample positions for placing sample tubes. By rotating the tray structure of the sample tray, the sample can be moved to a corresponding position, such as a position for the sample-dispensing mechanism 12 to draw the sample. The sample-dispensing mechanism 12 is used to draw the sample and dispense it into a reaction cup into which the sample should be added. For example, the sample-dispensing mechanism 12 may comprise a sample needle, which moves in two or three dimensions through a two-dimensional or three-dimensional driving mechanism. This allows the sample needle to move to draw the sample carried by the sample-carrying component 11, move to the reaction cup into which the sample should be added, and dispensing the sample into the reaction cup.

The reagent component 20 is used to carry reagents and, after drawing the reagents, provides them to the measurement component 30. In some embodiments, the reagent component 20 may comprise a reagent-carrying component 13 and a reagent-dispensing mechanism 14. The reagent-carrying component 13 is used to carry reagents. In one example, the reagent-carrying component 13 may be a reagent tray with a disc-shaped structure and multiple positions for carrying reagent containers. The reagent-carrying component 13 can rotate and rotate the reagent containers it carries, rotating them to a specific position, such as a position where the reagent-dispensing mechanism 14 draws the reagents. There may be one or more reagent-carrying components 13. The reagent-dispensing mechanism 14 is used to draw the reagents and dispense them into reaction cups into which the reagents should be added. In one example, the reagent-dispensing mechanism 14 may comprise a reagent needle, which moves in two or three dimensions through a two-dimensional or three-dimensional driving mechanism. This allows the needle to move to draw the reagents from the reagent-carrying component 13, move to the reaction cups into which the reagents should be added, and dispensing reagents into the reaction cups.

The measurement component 30 is used to test the analyte in the sample so as to obtain test data of the analyte. In some embodiments, the measurement component 30 may comprise a reaction component 15 and an optical measurement component 16. The reaction component 15 has at least one placement position for placing the reaction cup and incubating the reaction liquid within the reaction cup. For example, the reaction component 15 may be a reaction disk with a disc-shaped structure and one or more placement positions for placing the reaction cup. The reaction disk is rotatable, causing the reaction cup in its placement position to rotate, thus managing the reaction cup and incubating the reaction liquid within the reaction disk. The optical measurement component 16 is used to perform optical measurement on the reaction system obtained after mixing and during or after a predetermined time period to obtain the optical signal of the reaction system. For example, the optical measurement component 16 detects the intensity of transmitted and/or scattered light of the reaction system, and calculates the concentration of the analyte in the sample using a standard curve. In one example, the optical measurement component 16 is separately disposed outside the reaction component 15.

The following describes various examples of the sample analyzer of the present disclosure in detail with reference to FIGS. 3 to 5.

According to one example, the sample analyzer comprises: a sample component 10, a reagent component 20, a measurement component 30, and a controller 40.

The sample component 10 comprises a sample-carrying component 11 for carrying the sample to be tested and a sample-dispensing mechanism 12; the reagent component 20 comprises a reagent-carrying component 13 for carrying a reagent and a reagent-dispensing mechanism 14; the measurement component 30 comprises a reaction component 15 and an optical measurement component 16, wherein the reaction component 15 has at least one placement position for placing a reaction cup.

The controller 40 is configured to perform the following operations:
controlling the reagent-dispensing mechanism 14 to draw a first reagent and dispense it into the reaction cup, wherein the first reagent comprises a first carrier, the surface of which is bound with a first ligand capable of binding to an analyte in a sample to be tested; controlling the sample-dispensing mechanism 12 to draw the sample to be tested and dispense it into the reaction cup to obtain a first reaction system; controlling the first reaction system to react for a first predetermined time;
controlling the reagent-dispensing mechanism 14 to further draw a second reagent and dispense it into the reaction cup to obtain a second reaction system, wherein the second reagent comprises a second ligand capable of binding to the analyte; controlling the second reaction system to react for a second predetermined time;
controlling the optical measurement component 16 to acquire at least two optical signals;
obtaining a first reaction analysis value of the first reaction system based on at least one of the optical signals, and obtaining a second reaction analysis value of the second reaction system based on at least another of the optical signals; and
performing a determination based on the first reaction analysis value and/or the second reaction analysis value, selecting one of the first reaction analysis value and the second reaction analysis value according to the determination result to obtain an analysis result of the analyte in the sample to be tested, or selecting the optical signal corresponding to one of the first reaction analysis value and the second reaction analysis value according to the determination result to obtain an analysis result of the analyte in the sample to be tested.

The sample to be tested, the first reagent, and the second reagent are as described in the first aspect above, and will not be described again herein.

The sample analyzer of the present disclosure controls the reagent-dispensing mechanism 14 to first draw the first reagent, controls the sample-dispensing mechanism 12 to draw the sample to be tested and dispense it into a reaction cup for mixing to obtain a first reaction system and reacting for a first predetermined time, and then controls the reagent-dispensing mechanism 14 to further draw the second reagent and dispense it to the reaction cup for further mixing with the first reaction system and reacting for a second predetermined time. Similar to the sample analysis method described in the first aspect, controlling the optical measurement component 16 to acquire at least two optical signals can be performed simultaneously with controlling the sample-dispensing mechanism 12 to draw the sample and/or the reagent-dispensing mechanism 14 to draw the reagent, or it may also be conducted during the process of mixing and reacting the reagent and the sample in the reaction cup for a predetermined time, as well as after the end of the predetermined reaction time.

In some embodiments, controlling the optical measurement component 16 to acquire at least two optical signals comprises: acquiring at least one of the optical signals after controlling the reagent-dispensing mechanism 14 to draw the first reagent and dispense it to the reaction cup, and before controlling the reagent-dispensing mechanism 14 to further draw the second reagent and dispense it to the reaction cup; and acquiring at least another of the optical signals after controlling the reagent-dispensing mechanism 14 to further draw the second reagent and dispense it to the reaction cup.

In other embodiments, controlling the optical measurement component 16 to acquire at least two optical signals comprises: acquiring the at least two optical signals after controlling the reagent-dispensing mechanism 14 to further draw the second reagent and dispense it to the reaction cup.

In some embodiments, the controller 40 is configured to perform the following operations: performing a determination based on the first reaction analysis value and/or the second reaction analysis value, selecting one of the first reaction analysis value and the second reaction analysis value according to the determination result to obtain the analysis result of the analyte in the sample to be tested, or selecting the optical signal corresponding to one of the first reaction analysis value and the second reaction analysis value according to the determination result to obtain the analysis result of the analyte in the sample to be tested, comprising:
comparing the first reaction analysis value and the second reaction analysis value, selecting one of the first reaction analysis value and the second reaction analysis value according to the comparison result to obtain the analysis result of the analyte in the sample to be tested, or selecting the optical signal corresponding to one of the first reaction analysis value and the second reaction analysis value according to the comparison result to obtain the analysis result of the analyte in the sample to be tested.

In a preferred embodiment, the first reaction analysis value is compared with the second reaction analysis value, and the larger one between the first reaction analysis value and the second reaction analysis value is selected to obtain the analysis result of the analyte in the sample to be tested, or the optical signal corresponding to the larger one between the first reaction analysis value and the second reaction analysis value is selected to obtain the analysis result of the analyte in the sample to be tested.

According to other embodiments, it is determined whether the first reaction analysis value and/or the second reaction analysis value are within a preset range, and one of the first reaction analysis value and the second reaction analysis value is selected according to the determination result to obtain the analysis result of the analyte in the sample to be tested, or the optical signal corresponding to one of the first reaction analysis value and the second reaction analysis value is selected according to the determination result to obtain the analysis result of the analyte in the sample to be tested.

Another example of the second aspect of the present disclosure provides a sample analyzer, comprising: a sample component, a reagent component, a measurement component, and a controller.

The difference from the sample analyzer provided in the above example is that the controller is configured to control the optical measurement component to acquire at least two optical signals, and then perform the following operations:
obtaining a first reaction analysis value of the first reaction system based on at least one of the optical signals, and obtaining a second reaction analysis value of the second reaction system based on at least another of the optical signals; and obtaining an analysis result of the analyte in the sample to be tested based on the summation result of the first reaction analysis value and the second reaction analysis value.

The controller is configured to perform operations of ultimately obtaining the analysis result of the analyte in the sample to be tested through the at least two optical signals, which are the same as those described in the first aspect above, and will not be described again herein.

In the sample analyzers of the two examples described above, controlling the optical measurement component to acquire at least two optical signals comprises: acquiring at least one of the optical signals at a first detection wavelength, and acquiring at least another of the optical signals at a second detection wavelength.

Preferably, the first detection wavelength and the second detection wavelength are not the same.

According to some embodiments, the first detection wavelength and the second detection wavelength are selected from 320nm to 860nm; preferably, the first detection wavelength and the second detection wavelength are selected from 340nm to 850nm.

The following further describes some embodiments of controlling the optical measurement component to acquire at least two optical signals in the two examples described above.

According to a first embodiment, controlling the optical measurement component to acquire at least two optical signals comprises the following operations:
acquiring a first optical signal after controlling the reagent-dispensing mechanism to draw the first reagent and dispense it to the reaction cup, or, acquiring a first optical signal after controlling the reagent-dispensing mechanism to draw the first reagent and dispense it to the reaction cup and controlling the sample-dispensing mechanism to draw the sample to be tested and dispense it into the reaction cup, and before the start of the first predetermined time;
acquiring a second optical signal after the end of the first predetermined time and before controlling the reagent-dispensing mechanism to further draw the second reagent and dispense it to the reaction cup;
acquiring a third optical signal after controlling the reagent-dispensing mechanism to further draw the second reagent and dispense it to the reaction cup, and before the start of the second predetermined time;
acquiring a fourth optical signal after the end of the second predetermined time.

And "obtaining a first reaction analysis value of the first reaction system based on at least one of the optical signals, and obtaining a second reaction analysis value of the second reaction system based on at least another of the optical signals" comprising: obtaining a first reaction analysis value of the first reaction system based on the first optical signal and the second optical signal, and obtaining a second reaction analysis value of the second reaction system based on the third optical signal and the fourth optical signal.

According to a second embodiment, controlling the optical measurement component to acquire at least two optical signals comprises the following operations.
acquiring a second optical signal after the end of the first predetermined time and before controlling the reagent-dispensing mechanism to further draw the second reagent and dispense it to the reaction cup;
acquiring a fourth optical signal after the end of the second predetermined time.

And "obtaining a first reaction analysis value of the first reaction system based on at least one of the optical signals, and obtaining a second reaction analysis value of the second reaction system based on at least another of the optical signals" comprising: obtaining the first reaction analysis value of the first reaction system based on the second optical signal, and obtaining the second reaction analysis value of the second reaction system based on the fourth optical signal or based on the fourth optical signal and the second optical signal.

According to a third embodiment, controlling the optical measurement component to acquire at least two optical signals comprises the following operations:
acquiring a third optical signal after controlling the reagent-dispensing mechanism to further draw the second reagent and dispense it to the reaction cup and before the start of the second predetermined time;
acquiring a fourth optical signal after the end of the second predetermined time.

And "obtaining a first reaction analysis value of the first reaction system based on at least one of the optical signals, and obtaining a second reaction analysis value of the second reaction system based on at least another of the optical signals" comprising: obtaining the first reaction analysis value of the first reaction system based on the third optical signal, and obtaining the second reaction analysis value of the second reaction system based on the fourth optical signal or based on the fourth optical signal and third optical signal.

According to a fourth embodiment, controlling the optical measurement component to acquire at least two optical signals comprises the following operations:
acquiring a first optical signal after controlling the reagent-dispensing mechanism to draw the first reagent and dispense it to the reaction cup; or, acquiring a first optical signal after controlling the reagent-dispensing mechanism to draw the first reagent and dispense it to the reaction cup, and controlling the sample-dispensing mechanism to draw the sample to be tested and dispense it to the reaction cup, and before the start of the first predetermined time;
acquiring a second optical signal after the end of the first predetermined time and before controlling the reagent-dispensing mechanism to further draw the second reagent and dispense it to the reaction cup;
acquiring the fourth optical signal after the end of the second predetermined time.

And "obtaining a first reaction analysis value of the first reaction system based on at least one of the optical signals, and obtaining a second reaction analysis value of the second reaction system based on at least another of the optical signals" comprising: obtaining the first reaction analysis value of the first reaction system based on the first optical signal and the second optical signal, and obtaining the second reaction analysis value of the second reaction system based on the first optical signal and the fourth optical signal, or based on the second optical signal and the fourth optical signal.

According to a fifth embodiment, controlling the optical measurement component to acquire at least two optical signals comprises the following operations:
collecting multiple optical signals during at least a period from the start of the first predetermined time to the end of the first predetermined time, thereby acquiring a first reaction curve;
collecting multiple optical signals during at least a period from the start of the second predetermined time to the end of the second predetermined time, thereby acquiring a second reaction curve.

And "obtaining a first reaction analysis value of the first reaction system based on at least one of the optical signals, and obtaining a second reaction analysis value of the second reaction system based on at least another of the optical signals" comprising: obtaining the first reaction analysis value of the first reaction system based on the first reaction curve, and obtaining the second reaction analysis value of the second reaction system based on the second reaction curve.

In a preferred embodiment, the first reaction analysis value of the first reaction system is obtained based on the first reaction curve or based on a characteristic value of the first reaction curve, and the second reaction analysis value of the second reaction system is obtained based on the second reaction curve or based on a characteristic value of the second reaction curve.

The operations in the first to fifth embodiments of the sample analyzers described in the above two examples correspond to the steps in the first to fifth embodiments of the sample analysis method described in the first aspect above; therefore, the operations in each of the above embodiments will not be described again.

Similarly, corresponding to the sample analysis method described in the first aspect above, the operation modes of the sample analyzers disclosed herein are not limited to the above embodiments. Those skilled in the art can further obtain other operation modes of the sample analyzers based on the sample analysis methods described above.

Another example of the second aspect of the present disclosure provides a sample analyzer, comprising: a sample component 10, a reagent component 20, a measurement component 30, and a controller 40. Similar to the examples described above, the sample component 10 comprises a sample-carrying component 11 for carrying a sample to be tested and a sample-dispensing mechanism 12; the reagent component 20 comprises a reagent-carrying component 13 for carrying a reagent and a reagent-dispensing mechanism 14; the measurement component 30 comprises a reaction component 15 and an optical measurement component 16, wherein the reaction component 15 has at least one placement position for placing a reaction cup.

The controller 40 is configured to perform the following operations:
controlling the reagent-dispensing mechanism 14 to draw a first reagent and dispense it into the reaction cup, wherein the first reagent comprises a first carrier, the surface of which is bound with a first ligand capable of binding to an analyte in the sample to be tested; controlling the sample-dispensing mechanism 12 to draw the sample to be tested and dispense it into the reaction cup to obtain a first reaction system; controlling the first reaction system to react for a first predetermined time;
controlling the optical measurement component 16 to acquire at least one optical signal;
obtaining a first reaction analysis value of the first reaction system based on the at least one optical signal;
performing a determination based on the first reaction analysis value:
   when the determination result meets a preset condition, obtaining an analysis result of the analyte in the sample to be tested based on the first reaction analysis value;
   when the determination result does not meet the preset condition, controlling the reagent-dispensing mechanism 14 to further draw a second reagent and dispense it to the reaction cup to obtain a second reaction system, in which the second reagent comprises a second ligand capable of binding to the analyte, and controlling the second reaction system to react for a second predetermined time;
   controlling the optical measurement component 16 to acquire at least another optical signal;
   obtaining a second reaction analysis value of the second reaction system based on at least the at least another optical signal;
   obtaining an analysis result of the analyte in the sample to be tested based on at least the second reaction analysis value.

In this example, the controller 40 is configured to first control each component of the sample analyzer to perform operations of detecting the analyte in the sample to be tested using the first reagent, so that the first reagent is mixed with the sample to be tested to obtain a first reaction system and reacts for the first predetermined time, the at least one optical signal reflecting the reaction between the analyte in the sample to be tested and the first ligand in the first reagent is acquired, and the first reaction analysis value of the first reaction system is obtained based on the at least one optical signal. Then, the determination is performed based on the first reaction analysis value, and when the determination result meets the preset condition, the analysis result of the analyte in the sample to be tested is obtained based on the first reaction analysis value.

Thus, the sample analyzer of this example no longer needs to perform the operation of detection with the second reagent. Therefore, when the sample to be tested can be accurately detected using only the first reagent, the second reagent is unnecessary. Such a sample analyzer can save detection time and also save the second reagent.

When the judgment result does not meet the preset condition, the components of the sample analyzer are controlled to further add the second reagent to the reaction cup to obtain the second reaction system, and the second reaction system is controlled to react for the second predetermined time. During this process, the optical measurement component is controlled to acquire at least another optical signal.

According to some embodiments, the determination based on the first reaction analysis value comprises determining whether the first reaction analysis value is within a preset range.

According to one example, the controller 40 is configured to perform the following operations: controlling the reagent-dispensing mechanism 14 to draw a first reagent and dispense it to the reaction cup, in which the first reagent comprises a first carrier, the surface of which is bound with a first ligand capable of binding to an analyte in the sample to be tested; controlling the sample-dispensing mechanism 12 to draw the sample to be tested and dispense it to the reaction cup to obtain a first reaction system; controlling the reaction system of the first reaction system to react for a first predetermined time; controlling the optical measurement component 16 to acquire at least one optical signal; obtaining a first reaction analysis value of the first reaction system based on the at least one optical signal; performing a determination based on the first reaction analysis value, when the determination result meets a preset condition, obtaining an analysis result of the analyte in the sample to be tested based on the first reaction analysis value, when the determination result does not meet the preset condition, controlling the reagent-dispensing mechanism 14 to further draw a second reagent and dispense it to the reaction cup to obtain a second reaction system, in which the second reagent comprises a second carrier, the surface of which is bound with a second ligand capable of binding to the analyte in the sample to be tested, wherein the particle size of the first carrier is larger than the particle size of the second carrier; controlling the second reaction system to react for a second predetermined time; controlling the optical measurement component to acquire at least another optical signal; obtaining a second reaction analysis value of the second reaction system based on at least the at least another optical signal, and obtaining an analysis result of the analyte in the sample to be tested based on at least the second reaction analysis value.

According to some embodiments, obtaining the analysis result of the analyte in the sample to be tested based at least on the second reaction analysis value comprises: obtaining the analysis result of the analyte in the sample to be tested based on the second reaction analysis value.

According to other embodiments, obtaining the analysis result of the analyte in the sample to be tested based at least on the second reaction analysis value comprises: obtaining the analysis result of the analyte in the sample to be tested based on the first reaction analysis value and the second reaction analysis value. This embodiment is the same as the embodiments in the foregoing two examples where the analysis result of the analyte in the sample to be tested is obtained based on the first reaction analysis value and the second reaction analysis value. For example, the analysis result of the analyte in the sample to be tested can be obtained by performing a determination based on the first reaction analysis value and the second reaction analysis value, and then selecting one of them; or, the analysis result of the analyte in the sample to be tested is obtained by the summation result of the first reaction analysis value and the second reaction analysis value. Specific embodiments will not be described here.

Unlike the sample analyzers in the foregoing two example, in this example, the specific implementation mode of controlling the optical measurement component 16 to acquire at least one optical signal and obtaining the first reaction analysis value of the first reaction system based on the at least one optical signal may differ from the specific implementation mode of controlling the optical measurement component 16 to acquire at least another optical signal and obtaining the second reaction analysis value of the second reaction system based on at least another optical signal. That is, the at least one optical signal can be acquired and the first reaction analysis value of the first reaction system can be obtained based thereon using any one of the methods described in the first to fifth embodiments, and the at least another optical signal can be acquired and the second reaction analysis value of the second reaction system can be obtained based thereon using any one of the methods described in the first to fifth embodiments. For example, the first optical signal and the second optical signal of the first-stage reaction can be acquired using the fixed-point sampling method described in the first embodiment above, and the first reaction analysis value can be obtained thereby. Simultaneously, the second reaction curve can be acquired using the rate method described in the fifth embodiment above, and the second reaction analysis value can be obtained from the second reaction curve or a portion thereof.

In each of the embodiments, the optical signal can be a scattered optical signal and/or a transmitted optical signal.

The sample analyzers of the above examples may further comprise a user interface. According to some embodiments, the user interface is configured to display sample analysis modes for user selection and to activate corresponding analysis mode based on the user selection. According to some embodiments, the user interface is configured to display signal processing modes for user selection and to perform corresponding signal processing based on the user selection. According to some embodiments, the user interface is configured to output the analysis results of the analyte in the sample to be tested. The above embodiments can be combined in any way.

A third aspect of the present disclosure provides an immunoturbidimetric kit, comprising a first reagent and a second reagent. The first reagent comprises a first carrier with a first ligand bound to its surface; the second reagent comprises a second ligand; the first ligand and the second ligand may be the same or different, and are capable of binding to the same analyte in the same sample.

As previously described, the kit of the present disclosure comprises both the first ligand and the second ligand in the first reagent and the second reagent, respectively. That is, both of the reagents contain ligands capable of reacting with the analyte in the sample.

The second ligand in the second reagent may be in a free form or bound to the surface of the second carrier.

The first carrier and the second carrier may be the same or different. In a preferred embodiment, the particle size of the first carrier is larger than the particle size of the second carrier. However, the embodiments of the kit of the present disclosure is not limited to this. For example, the first carrier and the second carrier may be the same, or the particle size of the first carrier may be smaller than the particle size of the second carrier, as long as the first reagent is more suitable for detecting samples with lower analyte concentrations, and the second reagent is more suitable for detecting samples with higher analyte concentrations.

As previously described, the first reagent may comprise carriers with different particle sizes; the second reagent may also comprise carriers with different particle sizes, or further comprise a second ligand in a free form.

The carrier is preferably a latex microsphere, but is not limited thereto. For example, the carrier can also be magnetic beads, colloidal gold, silver nanoparticles, polymerized proteins, etc. In short, any substance that can induce a change in the optical signal in the reaction system after forming an aggregated state can be used as a carrier. Those skilled in the art can select according to actual needs.

According to some specific embodiments, the first carrier comprises a first latex microsphere, and the first latex microsphere has a particle size of 40nm to 500nm, preferably 80nm to 400nm.

In other embodiments, the second reagent does not contain a carrier, and the second ligand exists in a free form in the second reagent.

The kit disclosed in the present disclosure does not have any particular limitation on the type of ligand for each reagent.

According to some embodiments, the first ligand and the second ligand are the same. According to other embodiments, the binding affinity of the first ligand to the analyte is stronger than the binding affinity of the second ligand to the analyte.

According to some embodiments, the first ligand and the second ligand are antigens or antibodies capable of binding to the analyte in the sample to be tested. According to other embodiments, the first ligand and the second ligand can be substances capable of binding to the analyte in the sample, and the reaction products can alter the optical signal of the system and thus can be detected. For example, the first ligand and the second ligand can be aptamers, enzymes, or substrates. Those skilled in the art can select according to actual needs.

The selection of each ligand can be determined by those skilled in the art based on the actual analyte.

The amounts of the first ligand and the second ligand in the first reagent and the second reagent can also be determined by those skilled in the art based on the actual analyte.

In some embodiments, the first reagent further comprises a first buffer and an optional anti-interference agent; and the second reagent further comprises a second buffer.

The present disclosure does not particularly limit the type and pH value of the first and second buffers. Buffers conventionally used for detecting analytes in samples can be used, which can be selected and determined according to the specific sample and analyte.

The anti-interference agent can be a substance such as blocker or surfactant that eliminates, for example, rheumatoid factor or chylous interference. The present disclosure does not limit the type of anti-interference agent or its concentration in the first reagent. Conventional anti-interference agents and their dosages can be used, provided that the addition of the anti-interference agent to the first reagent does not affect the reaction and detection of the analyte in the sample to be tested.

If the anti-interference agent is unsuitable for addition to the first reagent, the kit further comprises a third reagent. According to one example, the third reagent is a pretreatment reagent, which comprises a third buffer and optionally an anti-interference agent. The third reagent can be used to pretreat the sample to be tested, for example, to dilute the sample and optionally eliminate interference.

The kit, with the three reagents, allows for more flexible treatment of the sample to be tested without adversely affecting detection, thus improving detection sensitivity and broadening the detection linear range.

The third buffer can also be any suitable buffer with any suitable pH value.

In other embodiments, the kit further comprises a fourth reagent and/or a fifth reagent.

The fourth reagent is similar to the first reagent, comprising a third ligand bound to the surface of a third microsphere. By selecting appropriate third microsphere and third ligand, the fourth reagent achieves relatively higher sensitivity, making it suitable for detecting samples with low analyte concentrations, thereby further broadening the detection lower limit.

The fifth reagent is similar to the second reagent, comprising a fourth ligand in free form or a fourth ligand bound to the surface of a fourth microsphere. By selecting appropriate fourth ligand, or fourth microsphere and fourth ligand, the fifth reagent achieves a relatively higher detection upper limit, making it suitable for detecting samples with high analyte concentrations, thereby further broadening the detection upper limit.

The following specific examples further illustrate the features and advantages of various aspects of the present disclosure.

### Examples

The reagents and sample analyzer used in the examples and comparative examples were as follows:
Reagents: Unless otherwise specified, all reagents were commercially available conventional reagents.
Sample Analyzer: Mindray BS-2800
Samples to be tested: Calibrators were self-made samples containing the analyte, calibrated using a standard detection system; clinical samples were collected samples; high-concentration samples were samples obtained by diluting pure products.

### Example 1. Detection of β2-MG concentration

Reagent R1 (i.e., the first reagent) and reagent R2 (i.e., the second reagent) were prepared according to the following formulations:
Reagent R1:

| | |
|---|---|
| 50mM Tris buffer | pH 7.5 |
| 180nm latex microspheres conjugated with β2-MG antibody | 40mg/L |

Reagent R2:

| | |
|---|---|
| 50mM Tris buffer | pH 7.5 |
| 100nm latex microspheres conjugated with β2-MG antibody | 320mg/L |

The series of standards A to H were tested using the sample analyzer, with water as a blank control (β2-MG concentrations are shown in Table 1 below). The specific method was as follows: 100 µL of reagent R1 was added to a reaction cup, followed by the addition of 2 µL of sample; 0.5 minutes after the addition was complete, blank absorbance S0L (i.e., the first optical signal) was read at a wavelength of 570nm; the mixture was then incubated at 37°C for 4 minutes, and absorbance S1L (i.e., the second optical signal) was read at a wavelength of 570nm. 100 µL of reagent R2 was added to the reaction cup; 0.5 minutes after the addition was complete, blank absorbance S0H (the third optical signal) was read at a wavelength of 570 nm; after the incubation was continued for approximately 5 minutes, the absorbance S1H (the fourth optical signal) was read at a wavelength of 570 nm. The units for absorbance readings and the signal values calculated from the absorbance readings were 0.0001 A; these units would not be mentioned again below.

Based on the absorbance readings, the low-concentration range signal value SL (the first reaction analysis value) and the high-concentration range signal value SH (the second reaction analysis value) were obtained: $Low - concentration range signal value SL = S 1 L - S 0 L ;$ $High - concentration range signal value SH = S 1 H - S 0 H .$

According to the first reaction analysis value and the second reaction analysis value, the summation result of the two (SL + SH) was obtained; and the two values were compared with the larger one (SL or SH) being selected. The detection results for each sample are shown in Table 1 below.

The two sets of signal values (SL and SH) obtained above were processed using the following three methods.

Method 1: Using multi-point fitting method (e.g., Spline), the functional relationship between the standard concentration and the signal value SL (calibration data 1), and the functional relationship between the calibrator concentration and the signal value SH (calibration data 2) were obtained, thus generating calibration curve 1 and calibration curve 2, respectively. See Table 2 below, which shows the relationships between concentrations and signal values. After obtaining the signal values SL and SH (i.e., the first reaction analysis value and the second reaction analysis value, hereinafter the same) from the sample to be tested, it was determined whether SL was less than 2098. If so, the concentration of β2-MG in the sample to be tested was obtained by applying SL into calibration curve 1; otherwise, the concentration of β2-MG in the sample to be tested was obtained by applying SH into calibration curve 2.

Method 2: Calibration curve 3 was obtained by using the summation result of the signal values SL and SH. The relationships between concentrations and signal values are shown in Table 2 below. After obtaining the signal values SL and SH from the sample to be tested, they also underwent summation, and the summation result was applied into calibration curve 3 to obtain the concentration of β2-MG in the sample to be tested.

Method 3: Calibration curve 4 was obtained by comparing the signal values SL and SH and taking the larger one of SL and SH. The relationships between concentrations and signal values are shown in Table 2. After obtaining the signal values SL and SH from the sample to be tested, they were also compared, and the larger one of SL and SH was applied into calibration curve 4 to obtain the concentration of β2-MG in the sample to be tested.

### Comparative Example 1. Detection of β2-MG concentration

Reagent R1 (i.e., buffer reagent) and reagent R2 were prepared according to the following formulations:
Reagent R1:

| | |
|---|---|
| 50mM Tris buffer | pH 7.5 |

Reagent R2:

| | |
|---|---|
| 50mM Tris buffer | pH 7.5 |
| 180nm latex microspheres conjugated with β2-MG antibody | 40mg/L |
| 100nm latex microspheres conjugated with β2-MG antibody | 320mg/L |

The series of standard samples were detected using the sample analyzer. The specific method was as follows: 100 µL of reagent R1 was added to the reaction cup, followed by the addition of 2 µL of sample, and incubated for 4 minutes, then 100 µL of reagent R2 was added. 0.5 minutes after the addition was complete, blank absorbance S0 was read at a wavelength of 570 nm. After the incubation was continued for 5 minutes, absorbance S1 was read at a wavelength of 570 nm.

The final signal value was obtained from the two absorbance readings: Signal value S = S1 - S0, as shown in Table 1.

Furthermore, based on the signal value S, the relationship between the concentration of a known concentration sample and the signal value S was established, thereby obtaining a standard curve. The correspondence between concentrations and signal values is shown in Table 2.

**Table 1. Detected signal values and processing results of signal values**

| Calibrator | Calibration Concentration (mg/L) | Signal Value (0.0001A) | | | | |
|---|---|---|---|---|---|---|
| | | Comparative Example 1 | Example 1 | | | |
| | | S | SL | SH | SL+SH | Larger of SL and SH |
| Water | 0 | -20 | -21 | -18 | -39 | -18 |
| A | 0.59 | 24 | 412 | 118 | 530 | 412 |
| B | 1.26 | 99 | 906 | 316 | 1222 | 906 |
| C | 2.39 | 242 | 1535 | 593 | 2128 | 1535 |
| D | 4.74 | 570 | 2098 | 954 | 3052 | 2098 |
| E | 9.43 | 1255 | 2430 | 1534 | 3963 | 2430 |
| F | 19.1 | 3227 | 2508 | 2645 | 5153 | 2645 |
| G | 34.14 | 6267 | 2305 | 5322 | 7628 | 5322 |
| H | 45.65 | 7945 | 2546 | 6675 | 9220 | 6675 |

**Table 2. Correspondence between concentrations and signal values (standard curve table)**

| Calibration concentration (mg/L) | Comparative Example 1 | Example 1 | | | | |
|---|---|---|---|---|---|---|
| | S | SL | SH | SL+SH | Larger of SL and SH | |
| | Calibration data | Calibration data 1 | Calibration data 2 | Calibration data 3 | Calibration data 4 | |
| | | | | | SL | SH |
| 0 | -20 | -21 | -18 | -39 | -18 | / |
| 0.59 | 24 | 412 | / | 530 | 412 | / |
| 1.26 | 99 | 906 | / | 1222 | 906 | / |
| 2.39 | 242 | 1535 | / | 2128 | 1535 | / |
| 4.74 | 570 | 2098 | 954 | 3052 | 2098 | / |
| 9.43 | 1255 | / | 1534 | 3963 | 2430 | / |
| 19.1 | 3227 | / | 2645 | 5153 | / | 2645 |
| 34.14 | 6267 | / | 5322 | 7628 | / | 5322 |
| 45.65 | 7945 | / | 6675 | 9220 | / | 6675 |

### Clinical testing:

Using the reagents of Example 1 and Comparative Example 1, 40 clinical samples were tested, respectively. Signal values SL, SH, and S were calculated according to the methods of Example 1 and Comparative Example 1, respectively. Signal values SL and SH were processed according to the three methods in Example 1, and the concentration values of each clinical sample were further obtained on the corresponding standard curves. For signal value S, the concentration values of each clinical sample were obtained on the corresponding standard curves according to the method in Comparative Example 1. Specific results are shown in Tables 3 and 4 below.

**Table 3. Signal values of each clinical sample and signal values processed according to the methods of Example 1 and Comparative Example 1**

| Sample No. | Comparative Example 1 | Example 1 | | | | | | |
|---|---|---|---|---|---|---|---|---|
| | Signal value | Signal value | | Signal value processed according to Method 1 | | Signal value processed according to Method 2 | Signal value processed according to Method 3 | |
| | S | SL | SH | SL | SH | SL+SH | SL | SH |
| 1 | 60 | 685 | 226 | 685 | / | 911 | 685 | / |
| 2 | 63 | 637 | 219 | 637 | / | 856 | 637 | / |
| 3 | 66 | 710 | 248 | 710 | / | 958 | 710 | / |
| 4 | 66 | 762 | 257 | 762 | / | 1020 | 762 | / |
| 5 | 70 | 719 | 248 | 719 | / | 967 | 719 | / |
| 6 | 73 | 788 | 271 | 788 | / | 1058 | 788 | / |
| 7 | 74 | 745 | 275 | 745 | / | 1020 | 745 | / |
| 8 | 76 | 785 | 270 | 785 | / | 1055 | 785 | / |
| 9 | 81 | 761 | 266 | 761 | / | 1027 | 761 | / |
| 10 | 90 | 880 | 307 | 880 | / | 1188 | 880 | / |
| 11 | 95 | 850 | 311 | 850 | / | 1160 | 850 | / |
| 12 | 98 | 932 | 342 | 932 | / | 1274 | 932 | / |
| 13 | 100 | 920 | 336 | 920 | / | 1256 | 920 | / |
| 14 | 100 | 906 | 326 | 906 | / | 1232 | 906 | / |
| 15 | 105 | 961 | 356 | 961 | / | 1317 | 961 | / |
| 16 | 109 | 1007 | 365 | 1007 | / | 1372 | 1007 | / |
| 17 | 110 | 998 | 354 | 998 | / | 1352 | 998 | / |
| 18 | 112 | 1021 | 365 | 1021 | / | 1385 | 1021 | / |
| 19 | 114 | 1043 | 373 | 1043 | / | 1416 | 1043 | / |
| 20 | 122 | 1059 | 411 | 1059 | / | 1470 | 1059 | / |
| 21 | 133 | 1101 | 404 | 1101 | / | 1506 | 1101 | / |
| 22 | 143 | 1179 | 439 | 1179 | / | 1618 | 1179 | / |
| 23 | 149 | 1162 | 448 | 1162 | / | 1610 | 1162 | / |
| 24 | 163 | 1287 | 490 | 1287 | / | 1777 | 1287 | / |
| 25 | 169 | 1319 | 517 | 1319 | / | 1836 | 1319 | / |
| 26 | 243 | 1590 | 653 | 1590 | / | 2243 | 1590 | / |
| 27 | 265 | 1653 | 683 | 1653 | / | 2337 | 1653 | / |
| 28 | 286 | 1677 | 713 | 1677 | / | 2389 | 1677 | / |
| 29 | 443 | 1956 | 914 | 1956 | / | 2871 | 1956 | / |
| 30 | 511 | 2052 | 994 | 2052 | / | 3045 | 2052 | / |
| 31 | 1185 | 2416 | 1444 | / | 1444 | 3860 | 2416 | / |
| 32 | 2975 | 2569 | 2581 | / | 471 | 5149 | / | 2581 |
| 33 | 3565 | 2405 | 2970 | / | 307 | 5375 | / | 2970 |
| 34 | 3599 | 2456 | 3043 | / | 358 | 5499 | / | 3043 |
| 35 | 4231 | 2378 | 3352 | / | 280 | 5729 | / | 3352 |
| 36 | 4349 | 2366 | 3545 | / | 268 | 5911 | / | 3545 |
| 37 | 5118 | 2340 | 4252 | / | 242 | 6592 | / | 4252 |
| 38 | 5791 | 2329 | 4962 | / | 231 | 7291 | / | 4962 |
| 39 | 6771 | 2352 | 5803 | / | 254 | 8155 | / | 5803 |
| 40 | 7791 | 2474 | 6640 | / | 376 | 9115 | / | 6640 |

**Table 4. Concentrations and relative deviations of each clinical sample obtained based on signal values or signal values processed according to the methods of Example 1**

| Sample No. | Comparative Example 1 | Example 1 | | | | | |
|---|---|---|---|---|---|---|---|
| | | Method 1 | | Method 2 | | Method 3 | |
| | Concentration (mg/L) | Concentration (mg/L) | Relative deviation (%) | Concentration (mg/L) | Relative deviation (%) | Concentration (mg/L) | Relative deviation (%) |
| 1 | 0.94 | 0.95 | 1.49 | 0.96 | 1.71 | 0.95 | 1.47 |
| 2 | 0.96 | 0.89 | -7.59 | 0.90 | -6.11 | 0.89 | -7.60 |
| 3 | 0.98 | 0.99 | 0.32 | 1.00 | 1.75 | 0.99 | 0.30 |
| 4 | 0.99 | 1.06 | 6.74 | 1.06 | 6.91 | 1.06 | 6.71 |
| 5 | 1.02 | 1.00 | -2.18 | 1.01 | -1.22 | 1.00 | -2.21 |
| 6 | 1.04 | 1.09 | 4.82 | 1.10 | 5.25 | 1.09 | 4.79 |
| 7 | 1.05 | 1.04 | -1.74 | 1.06 | 0.71 | 1.04 | -1.77 |
| 8 | 1.07 | 1.09 | 1.57 | 1.09 | 1.99 | 1.09 | 1.54 |
| 9 | 1.11 | 1.06 | -5.03 | 1.07 | -4.06 | 1.06 | -5.06 |
| 10 | 1.19 | 1.22 | 3.22 | 1.23 | 3.40 | 1.22 | 3.21 |
| 11 | 1.23 | 1.18 | -4.19 | 1.20 | -2.69 | 1.18 | -4.21 |
| 12 | 1.25 | 1.30 | 3.85 | 1.31 | 5.05 | 1.30 | 3.86 |
| 13 | 1.26 | 1.28 | 1.22 | 1.30 | 2.38 | 1.28 | 1.23 |
| 14 | 1.27 | 1.26 | -0.41 | 1.27 | 0.41 | 1.26 | -0.41 |
| 15 | 1.31 | 1.34 | 2.61 | 1.36 | 3.99 | 1.34 | 2.64 |
| 16 | 1.34 | 1.41 | 4.79 | 1.42 | 5.37 | 1.41 | 4.87 |
| 17 | 1.35 | 1.40 | 3.67 | 1.40 | 3.61 | 1.40 | 3.74 |
| 18 | 1.36 | 1.43 | 4.95 | 1.43 | 5.01 | 1.43 | 5.04 |
| 19 | 1.38 | 1.46 | 6.16 | 1.46 | 6.16 | 1.47 | 6.27 |
| 20 | 1.44 | 1.49 | 3.08 | 1.52 | 5.50 | 1.49 | 3.21 |
| 21 | 1.54 | 1.56 | 1.16 | 1.56 | 1.66 | 1.56 | 1.34 |
| 22 | 1.61 | 1.68 | 4.26 | 1.69 | 4.97 | 1.69 | 4.53 |
| 23 | 1.66 | 1.65 | -0.36 | 1.68 | 1.45 | 1.66 | -0.12 |
| 24 | 1.78 | 1.87 | 5.34 | 1.89 | 6.36 | 1.88 | 5.70 |
| 25 | 1.82 | 1.93 | 5.89 | 1.97 | 7.88 | 1.94 | 6.26 |
| 26 | 2.40 | 2.53 | 5.50 | 2.58 | 7.52 | 2.52 | 5.13 |
| 27 | 2.56 | 2.72 | 5.97 | 2.75 | 7.31 | 2.69 | 4.95 |
| 28 | 2.72 | 2.79 | 2.65 | 2.85 | 4.90 | 2.75 | 1.35 |
| 29 | 3.85 | 4.05 | 5.20 | 4.08 | 6.00 | 3.81 | -1.12 |
| 30 | 4.32 | 4.54 | 4.93 | 4.71 | 9.05 | 4.38 | 1.42 |
| 31 | 8.99 | 8.53 | -5.10 | 8.76 | -2.63 | 9.22 | 2.59 |
| 32 | 17.97 | 18.66 | 3.85 | 19.07 | 6.13 | 18.72 | 4.16 |
| 33 | 20.62 | 21.13 | 2.45 | 20.68 | 0.30 | 20.95 | 1.60 |
| 34 | 20.78 | 21.55 | 3.73 | 21.51 | 3.53 | 21.35 | 2.77 |
| 35 | 23.67 | 23.28 | -1.62 | 22.97 | -2.93 | 23.03 | -2.71 |
| 36 | 24.22 | 24.32 | 0.42 | 24.08 | -0.59 | 24.06 | -0.68 |
| 37 | 27.92 | 28.02 | 0.37 | 28.02 | 0.36 | 27.81 | -0.39 |
| 38 | 31.42 | 31.91 | 1.57 | 32.07 | 2.07 | 31.83 | 1.32 |
| 39 | 37.37 | 37.77 | 1.06 | 37.73 | 0.95 | 37.83 | 1.23 |
| 40 | 44.58 | 45.35 | 1.74 | 44.88 | 0.68 | 45.35 | 1.74 |

As can be seen from Tables 3 to 4 above regarding the detection of clinical samples, all methods described in Example 1 for processing signal values could obtain accurate detection results. The deviations relative to the control examples were all within 10%, showing good consistency.

### Reproducibility of low-concentration samples:

Samples with β2-MG concentrations of 0.2 mg/L, 1.0 mg/L, and 20 mg/L were subjected to 20 parallel tests using the above methods. The detection concentrations of different methods were obtained according to Comparative Example 1 and the three methods described in Example 1, and the mean, standard deviation (SD), and coefficient of variation (CV) were calculated, as shown in Table 5 below.

**Table 5. Detection results of samples to be tested**

| No. | Concentration of sample to be tested (mg/L) | Item | Comparative Example 1 | Example 1 | | |
|---|---|---|---|---|---|---|
| | | | | Method 1 | Method 2 | Method 3 |
| 1 | 0.2 | Mean concentration (mg/L) | 0.05 | 0.12 | 0.11 | 0.12 |
| | | SD | 0.03 | 0.00 | 0.00 | 0.00 |
| | | CV | 68.91% | 2.72% | 3.66% | 2.80% |
| 2 | 1.0 | Mean concentration (mg/L) | 1.05 | 1.04 | 1.05 | 1.04 |
| | | SD | 0.04 | 0.02 | 0.03 | 0.02 |
| | | CV | 3.43% | 1.96% | 2.46% | 1.99% |
| 3 | 20.0 | Mean concentration (mg/L) | 21.00 | 21.45 | 21.31 | 21.48 |
| | | SD | 0.50 | 0.22 | 0.49 | 0.33 |
| | | CV | 2.38% | 1.03% | 2.31% | 1.56% |

As can be seen from the above coefficient of variation (CV), the methods in Example 1 had better accuracy and precision, especially with significantly improved sensitivity at low concentrations, thus broadening the linear detection range.

### Example 2. Detection of β2-MG

Reagent R1 (i.e., the first reagent) and reagent R2 (i.e., the second reagent) were prepared according to the following formulations:
Reagent R1:

| | |
|---|---|
| 25mM phosphate buffer | pH 7.4 |
| 190nm latex microspheres conjugated with rabbit anti-human β2-MG antibody | 50mg/L |
| Blocker (rabbit IgG) | 1g/L |

Reagent R2:

| | |
|---|---|
| 25mM phosphate buffer | pH 7.5 |
| 80nm latex microspheres conjugated with goat anti-human β2-MG antibody | 800mg/L |

The samples were detected using the sample analyzer. The specific method was as follows: 100 µL of reagent R1 was added to the reaction cup, followed by the addition of 2 µL of sample; 0.5 minutes after the addition was complete, blank absorbance S0L (i.e., the first optical signal) was read at a wavelength of 570 nm; after incubation was continued at 37°C for 4 minutes, absorbance S1L (i.e., the second optical signal) was read; then, 100 µL of reagent R2 was added to the reaction cup; 0.5 minutes after the addition was complete, blank absorbance S0H (i.e., the third optical signal) was read at a wavelength of 570 nm; after incubation was continued for 5 minutes, absorbance S1H (i.e., the fourth optical signal) was read at a wavelength of 570 nm.

Based on these absorbance readings, the low-concentration range signal value SL (i.e., the first reaction analysis value) and the high-concentration range signal value SH (i.e., the second reaction analysis value) were obtained, respectively: $Low - concentration range signal value SL = S 1 L - S 0 L ;$ $High - concentration range signal value SH = S 1 H - S 0 H .$

The first reaction analysis value SL was compared with three times the second reaction analysis value 3*SH. When SL > 3*SH, SL was used as the signal value of the output result; otherwise, SH was used as the signal value of the output result. The detection results for each sample are shown in Table 6 below.

Furthermore, based on the selected signal value (SL or SH), the concentration of β2-MG in each sample was obtained from the corresponding calibration curve (the correspondence between signal value and concentration was established using calibrators).

### Example 3. Detection of β2-MG

The same samples were detected using the first reagent and the second reagent of Example 3. Specifically, 100 µL of reagent R1 was added to the reaction cup, followed by the addition of 2 µL of sample; immediately at 0.5 minutes after the addition was complete, blank absorbance S0L (i.e., the first optical signal) was read at a wavelength of 412 nm; after incubation was continued at 37°C for 4 minutes, absorbance S1L (i.e., the second optical signal) was read. 100 µL of reagent R2 was added to the reaction cup; immediately at 0.5 minutes after the addition was complete, blank absorbance S0H (i.e., the third optical signal) was read at a wavelength of 605 nm. After incubation was continued for 5 minutes, absorbance S1H (i.e., the fourth optical signal) was further read.

Based on the above absorbance readings, the low-concentration range signal value SL (i.e., the first reaction analysis value) and the high-concentration range signal value SH (i.e., the second reaction analysis value) were obtained, respectively: $Low - concentration range signal value SL = S 1 L - S 0 L ;$ $High - concentration range signal value SH = S 1 H - S 0 H .$

The first reaction analysis value SL was compared with three times the second reaction analysis value 3*SH. If SL > 3*SH, SL was used as the signal value of the output result; otherwise, SH was used as the signal value of the output result. The detection results for each sample are shown in Table 6 below.

Further, based on the selected signal value (SL or SH), the concentration of β2-MG in each sample was obtained from the corresponding calibration curve (the correspondence between signal value and concentration was established using calibrators).

### Example 4. Detection of β2-MG

The same samples were detected using the first reagent and the second reagent of Example 3. Specifically, 100 µL of reagent R1 was added to the reaction cup, followed by the addition of 2 µL of the sample; after mixing, the mixture was incubated at 37°C for 4 minutes, during which the absorbance was continuously read at a wavelength of 570 nm to obtain the first reaction curve. Then, 100 µL of reagent R2 was added to the reaction cup, and the mixture was incubated at 37°C for 5 minutes, during which the absorbance was continuously read at a wavelength of 570 nm to obtain the second reaction curve.

Absorbance values S₁₁ and S₁₂ were obtained at an interval of 18 seconds around the 2-minute mark of the first reaction curve, and the rate of change of absorbance per minute was calculated as SL = (S₁₂ - S₁₁)/(18*60) (i.e., the first reaction analysis value). Absorbance values S₂₁ and S₂₂ were obtained at an interval of 18 seconds around 1.5-minute mark of the second reaction curve, and the rate of change of absorbance per minute was calculated as SH = (S₂₁ - S₂₂)/(18*60) (i.e., the first reaction analysis value).

The first reaction analysis value SL was compared with 2.8 times the second reaction analysis value, 2.8*SH. When SL > 2.8*SH, SL was used as the signal value of the output result; otherwise, SH was used as the signal value of the output result. The detection results for each sample are shown in Table 6 below.

Furthermore, based on the selected signal value (SL or SH), the concentration of β2-MG in each sample was obtained from the corresponding calibration curve (the correspondence between signal value and concentration was established using calibrators).

### Example 5. Detection of β2-MG

The same samples were detected using the first reagent and the second reagent of Example 3. The specific method was as follows: 100 µL of reagent R1 was added to the reaction cup, then 2 µL of the sample was added; after mixing, the mixture was incubated at 37°C for 4 minutes, during which the absorbance was continuously read at a wavelength of 570 nm to obtain the first reaction curve. Then, 100 µL of reagent R2 was added to the reaction cup, mixed, and incubated at 37°C for 5 minutes, during which the absorbance was continuously read at a wavelength of 570 nm to obtain the second reaction curve.

On the first reaction curve, the difference in absorbance between two adjacent time points, Sn and Sn-1, at an interval of 18 seconds was calculated. n was the ordinal number of the monitoring point from the time of mixing reagent R1 and the sample to the time of adding reagent R2. In this example, 5 < n < 16, and n was a natural number. The absorbance change rate per minute at each monitoring point was calculated as (Sn - Sn-1) / (18*60), and SL = MAX[(Sn - Sn-1)/(18*60)] (i.e., the first reaction analysis value) was taken. On the second reaction curve, the difference in absorbance between two adjacent time points, Sn and Sn-1, at an interval of 18 seconds was calculated. n was the ordinal number of the monitoring point from the addition of reagent R2 to the end of incubation; in this example, 18 < n < 33, and n was a natural number. The absorbance change rate per minute at each monitoring point was calculated as (Sn - Sn-1)/(18*60), and SH = MAX[(Sn - Sn-1)/(18*60)] (i.e., the second reaction analysis value) was taken.

The first reaction analysis value SL was compared with the second reaction analysis value SH. When SL > SH, SL was used as the signal value of the output result; otherwise, SH was used as the signal value of the output result. The detection results for each sample are shown in Table 6 below.

Furthermore, based on the selected signal value (SL or SH), the concentration of β2-MG in each sample was obtained from the corresponding calibration curve (the correspondence between signal value and concentration was established using calibrators).

### Comparative Example 2. Detection of β2-MG

Reagent R1 (i.e., buffer reagent) and reagent R2 were prepared according to the following formulations:
Reagent R1:

| | |
|---|---|
| 50mM Tris buffer | pH 7.5 |

Reagent R2:

| | |
|---|---|
| 50mM Tris buffer | pH 7.5 |
| 210nm latex microspheres conjugated with β2-MG antibody | 100mg/L |
| 800nm latex microspheres conjugated with β2-MG antibody | 800mg/L |

The samples were analyzed using the sample analyzer. The specific method was as follows: 100 µL of reagent R1 was added to the reaction cup, followed by the addition of 2 µL of the sample, and incubated at 37°C for 4 minutes, and then 100 µL of reagent R2 was added. Immediately at 0.5 minutes after the addition was complete, blank absorbance S0 was read at a wavelength of 570nm. The incubation was continued at 37°C for 5 minutes, and then absorbance S1 was read at a wavelength of 570nm.

The final signal value was obtained based on the two absorbance readings: Signal value S = S1 - S0, as shown in Table 6.

Further, based on the signal value S, the concentration of β2-MG in each sample was obtained from the calibration curve (the correspondence between signal value and concentration was established using calibrators).

**Table 6**

| No. | Sample concentration (mg/L) | Signal value | | | | | | | | |
|---|---|---|---|---|---|---|---|---|---|---|
| | | Comparative Example 2 | Example 2 | | Example 3 | | Example 4 | | Example 5 | |
| | | | SL | SH | SL | SH | SL | SH | SL | SH |
| 1 | 0 | -27.80 | **-50** | -18 | **-78** | -24 | **-17** | -6 | **-12** | 8 |
| 2 | 0.3125 | -22.13 | **325** | 34 | **466** | 31 | **112** | -36 | **117** | 99 |
| 3 | 0.625 | -0.47 | **662** | 114 | **902** | 102 | **233** | 43 | **233** | 113 |
| 4 | 1.25 | 93.87 | **1607** | 413 | **1963** | **380** | **520** | **177** | **602** | **377** |
| 5 | 2.5 | 187.58 | **1988** | **624** | 2280 | **572** | 605 | **258** | 815 | **691** |
| 6 | 5 | 475.16 | 2232 | **901** | 2411 | **831** | 622 | **352** | 1035 | **982** |
| 7 | 10 | 1053.59 | 2307 | **1310** | 2390 | **1213** | 608 | **421** | 1188 | **1329** |
| 8 | 20 | 2377.53 | 2302 | **2326** | 2311 | **2145** | 595 | **771** | 1262 | **2108** |
| 9 | 40.00 | 6521.56 | 2203 | **6001** | 2181 | **5585** | 575 | **1848** | 1228 | **3115** |
| 10 | 80.00 | 8620.29 | 2147 | **8107** | 2141 | **7582** | 554 | **2466** | 1181 | **3663** |

Generally speaking, for the same low-concentration sample, a higher signal value (after subtracting the signal at sample concentration of 0) reflects a higher sensitivity of the method used; for high-concentration samples, a continuously increase in signal value as the concentration increases (i.e., a monotonically increasing concentration-signal curve) reflects a higher detection upper limit of the method used. As shown in the table above, the detection sensitivity of each example was significantly improved compared to Comparative Example 2, thereby increasing the overall detection range.

### Example 6. Detection of β2-MG

Reagent R1 (i.e., the first reagent) and reagent R2 (i.e., the second reagent) were prepared according to the following formulations:
Reagent R1:

| | |
|---|---|
| 50mM Tris buffer | pH 7.5 |
| 100nm latex microspheres conjugated with β2-MG antibody | 200mg/L |

Reagent R2:

| | |
|---|---|
| 50mM Tris buffer | pH 7.5 |
| 100nm latex microspheres conjugated with β2-MG antibody | 200mg/L |

### Determination of optimal detection wavelength:

Using multiple detection wavelengths ranging from 340nm to 850nm, the absorbance of a sample containing approximately 0.375mg/L of β2-MG was measured 20 times repeatedly. The mean, standard deviation (SD), and coefficient of variation (CV) for each wavelength were calculated, as shown in Table 7 below.

**Table 7**

| Detection wavelength (nm) | Absorbance (A) | | |
|---|---|---|---|
| | Mean | Standard deviation (SD) | Coefficient of variation (CV) |
| 340 | 298.89 | 61.83% | 20.69% |
| 380 | 228.49 | 16.10% | 7.04% |
| 412 | 188.73 | 8.67% | 4.60% |
| 450 | 151.83 | 8.89% | 5.86% |
| 505 | 113.69 | 9.15% | 8.05% |
| 546 | 93.98 | 9.82% | 10.45% |
| 570 | 84.53 | 9.39% | 11.11% |
| 605 | 73.04 | 9.25% | 12.67% |
| 660 | 57.98 | 9.14% | 15.77% |
| 700 | 49.36 | 8.77% | 17.76% |
| 740 | 42.38 | 8.46% | 19.96% |
| 800 | 33.50 | 8.28% | 24.73% |
| 850 | 28.55 | 8.03% | 28.12% |

According to Table 7 above, 412 nm, with the smallest coefficient of variation, was used as the detection wavelength in the low concentration range after adding the first reagent. For the detection wavelength after further adding the second reagent, a larger wavelength was used for high-concentration samples to avoid exceeding the instrument's detection upper limit; therefore, 570 nm was used as the detection wavelength.

The samples were detected tested using the sample analyzer. The specific method was as follows: 100 µL of reagent R1 was added to the reaction cup, followed by the addition of 2 µL of the sample, and immediately at 0.5 minutes after the addition was complete, blank absorbance S0L (i.e., the first optical signal) was read at a wavelength of 412 nm. After the incubation was continued at 37°C for approximately 4 minutes, absorbance S1L (i.e., the second optical signal) was read. Then, 100 µL of reagent R2 was added to the reaction cup, and immediately at 0.5 minutes after the addition was complete, blank absorbance S0H (i.e., the third optical signal) was read at a wavelength of 505 nm. After the incubation was continued for approximately 5 minutes, absorbance S1H (i.e., the fourth optical signal) was read at a wavelength of 570 nm.

Based on the above absorbance readings, the low-concentration range signal value SL (i.e., the first reaction analysis value) and the high-concentration range signal value SH (i.e., the second reaction analysis value) were obtained, respectively: $Low - concentration range signal value SL = S 1 L - S 0 L ;$ $High - concentration range signal value SH = S 1 H - S 0 H .$

The first reaction analysis value SL was compared with signal threshold 6000. If SL < 6000, SL was used as the signal value of the output result; otherwise, SH was used as the signal value of the output result. The detection results for each sample are shown in Table 8 below.

Furthermore, based on the selected signal value (SL or SH), the concentration of β2-MG in each sample could be obtained from the corresponding calibration curve (the correspondence between signal value and concentration was established using calibrators).

### Comparative Example 3. Detection of β2-MG

Reagent R1 (i.e., buffer reagent) and reagent R2 were prepared according to the following formulations:
Reagent R1:

| | |
|---|---|
| 50mM Tris buffer | pH 7.5 |

Reagent R2:

| | |
|---|---|
| 50mM Tris buffer | pH 7.5 |
| 100nm latex microspheres conjugated with β2-MG antibody | 1g/L |

The samples were detected using the sample analyzer. The specific method was as follows: 160 µL of reagent R1 was added to the reaction cup, then 2 µL of the sample was added, and incubated at 37°C for 4 minutes. Then, 40 µL of reagent R2 was added, and immediately at 0.5 minutes after the addition was complete, blank absorbance S0 was read at a wavelength of 450 nm. The incubation was continued at 37°C for 5 minutes, and then absorbance S1 was read at a wavelength of 450 nm. (Note: At the optimal detection wavelength of 412 nm determined in the above examples, high-concentration samples exceeded the instrument's detection limit; therefore, 450 nm was selected for detection.)

The final signal value obtained based on the two absorbance readings was: Signal value S = S1 - S0, as shown in Table 8.

Further, based on the signal value S, the concentration of β2-MG in each sample was obtained from the calibration curve (the correspondence between signal value and concentration was established using calibrators).

**Table 8**

| No. | Sample concentration (mg/L) | Comparative Example 3 | Example 6 | |
|---|---|---|---|---|
| | | S | SL | SH |
| 1 | 0.00 | 29 | **-11** | -3 |
| 2 | 0.38 | 135 | **239** | 31 |
| 3 | 0.75 | 268 | **523** | 34 |
| 4 | 1.50 | 584 | **1219** | 111 |
| 5 | 3.00 | 1410 | **2864** | 349 |
| 6 | 6.00 | 3197 | **5661** | 821 |
| 7 | 12.00 | 6282 | 9173 | **1850** |
| 8 | 25.00 | 9660 | 11531 | **3686** |
| 9 | 50.00 | 11644 | 11801 | **5321** |
| 10 | 100.00 | 11536 | 11389 | **5785** |

### Example 7. Detection of CRP

Reagent R1 (i.e., the first reagent) and reagent R2 (i.e., the second reagent) were prepared according to the following formulations.
Reagent R1:

| | |
|---|---|
| 20mM phosphate buffer | pH 7.5 |
| 210nm latex microspheres conjugated with CRP antibody | 100mg/L |

Reagent R2:

| | |
|---|---|
| 20mM phosphate buffer | pH 7.5 |
| 60nm latex microspheres conjugated with CRP antibody | 600mg/L |

The samples were detected using the sample analyzer. The specific method was as follows: 180 µL of reagent R1 was added to the reaction cup, followed by the addition of 3.6 µL of sample, and immediately at 0.5 minutes after the addition was complete, blank absorbance S0L (i.e., the first optical signal) was read at a wavelength of 570 nm. After the incubation was continued at 37°C for 4 minutes, absorbance S1L (i.e., the second optical signal) was read at a wavelength of 570 nm. Then, 60 µL of reagent R2 was added to the reaction cup, mixed, and incubated at 37°C for 5 minutes, and then absorbance S1H (i.e., the fourth optical signal) was read at a wavelength of 570 nm.

Based on the above absorbance readings, the low-concentration range signal value SL (i.e., the first reaction analysis value) and the high-concentration range signal value SH (i.e., the second reaction analysis value) were obtained, respectively: $Low - concentration range signal value SL = S 1 L - S 0 L ;$ $High - concentration range signal value SH = S 1 H - S 0 L .$

Specific signal values are shown in Table 9.

### Example 8. Detection of CRP

Reagents were prepared according to the formulation in Example 7, and the samples were detected using the sample analyzer. The specific method was as follows: 180 µL of reagent R1 was added to the reaction cup, then 3.6 µL of the sample was added and mixed, and immediately at 0.5 minutes after the addition was complete, blank absorbance S0L (i.e., the first optical signal) was read at a wavelength of 450 nm. After the incubation was continued at 37°C for 4 minutes, absorbance S1L (i.e., the second optical signal) was read at 450 nm wavelength, and absorbance S0H (i.e., the third optical signal) was read at a wavelength of 700 nm. Then, 60 µL of reagent R2 was added to the reaction cup and mixed well, and incubated at 37°C for 5 minutes, and absorbance S1H (i.e., the fourth optical signal) was read at a wavelength of 700 nm.

Based on the above absorbance readings, the low-concentration range signal value SL (i.e., the first reaction analysis value) and the high-concentration range signal value SH (i.e., the second reaction analysis value) were obtained, respectively: $Low - concentration range signal value SL = S 1 L - S 0 L ;$ $High - concentration range signal value SH = S 1 H - S 0 H .$

Specific signal values are shown in Table 9.

### Example 9. Detection of CRP

Reagents were prepared according to the formulation in Example 7, and the samples were detected using the sample analyzer. Specifically, 80 µL of reagent R1 was added to the reaction cup, followed by the addition of 3.6 µL of sample and mixed, and immediately at 0.5 minutes after the addition was complete, blank absorbance S0L (i.e., the first optical signal) was read at a wavelength of 570 nm. After the incubation was performed at 37°C for 4 minutes, absorbance S1L (i.e., the second optical signal) was read at a wavelength of 570 nm. Then, 160 µL of reagent R2 was added to the reaction cup, mixed thoroughly, and immediately at 0.5 minutes after the addition was complete, blank absorbance S0H (i.e., the third optical signal) was read at a wavelength of 570 nm. After the incubation was performed at 37°C for 5 minutes, absorbance S1H (i.e., the fourth optical signal) was read at a wavelength of 570 nm.

Based on the above absorbance readings, the low-concentration range signal value SL (i.e., the first reaction analysis value) and the high-concentration range signal value SH (i.e., the second reaction analysis value) were obtained, respectively: $Low - concentration range signal value SL = S 1 L - S 0 L ;$ $High - concentration range signal value SH = S 1 H - S 0 H .$

Specific signal values are shown in Table 9.

### Example 10. Detection of CRP

Reagent R1 (i.e., the first reagent) and reagent R2 (i.e., the second reagent) were prepared according to the following formulation. Compared with Example 7, the content of latex microspheres in reagent R2 was reduced by 62.5%.
Reagent R1:

| | |
|---|---|
| 20mM phosphate buffer | pH 7.5 |
| 210nm latex microspheres conjugated with CRP antibody | 100mg/L |

Reagent R2:

| | |
|---|---|
| 20mM phosphate buffer | pH 7.5 |
| 60nm latex microspheres conjugated with CRP antibody | 225mg/L |

The samples were detected using the sample analyzer. The specific method was as follows: 80 µL of reagent R1 was added to the reaction cup, followed by the addition of 1.6 µL of the sample, and immediately at 0.5 minutes after the addition was complete, blank absorbance S0L (i.e., the first optical signal) was read at a wavelength of 570 nm. After the incubation was performed at 37°C for 4 minutes, absorbance S1L (i.e., the second optical signal) was read at a wavelength of 570 nm. Then, 160 µL of reagent R2 was added to the reaction cup, mixed well, and immediately at 0.5 minutes after the addition was complete, blank absorbance S0H (i.e., the third optical signal) was read at a wavelength of 570 nm. The incubation was continued at 37°C for 5 minutes, and absorbance S1H (i.e., the fourth optical signal) was read at a wavelength of 570 nm.

Based on the above absorbance readings, the low-concentration range signal value SL (i.e., the first reaction analysis value) and the high-concentration range signal value SH (i.e., the second reaction analysis value) were obtained, respectively: $Low - concentration range signal value SL = S 1 L - S 0 L ;$ $High - concentration range signal value SH = S 1 H - S 0 L .$

Specific signal values are shown in Table 9.

### Comparative Example 4. Detection of CRP

Reagents were prepared according to the formulation of Example 7, and the samples were detected using the sample analyzer. The specific method was as follows: 180 µL of reagent R1 was added to the reaction cup, then 3.6 µL of the sample was added and mixed, and immediately at 0.5 minutes after the addition was complete, blank absorbance S0 (i.e., the first optical signal) was read at a wavelength of 570 nm. The incubation was continued at 37°C for 4 minutes, and then 60 µL of reagent R2 was added to the reaction cup, and mixed well. After the mixture was incubated at 37°C for 5 minutes, absorbance S1 (i.e., the fourth optical signal) was read at a wavelength of 570 nm.

The signal value S was obtained based on the above absorbance readings: $Signal value S = S 1 - S 0 .$

Specific signal values are shown in Table 9.

**Table 9**

| No. | Sample concentration (mg/L) | Comparative Example 4 | Example 7 | | Example 8 | | Example 9 | | Example 10 | |
|---|---|---|---|---|---|---|---|---|---|---|
| | | S | SL | SH | SL | SH | SL | SH | SL | SH |
| 1 | 0 | **-388** | **7** | -395 | **13** | -377 | **9** | 45 | **11** | 29 |
| 2 | 0.49 | **-290** | **90** | -380 | **139** | -365 | **257** | 60 | **100** | 21 |
| 3 | 0.98 | **-69** | **284** | -353 | **437** | -349 | **555** | 82 | **264** | 39 |
| 4 | 1.95 | **315** | **609** | -294 | **946** | -308 | **1104** | 123 | **600** | 71 |
| 5 | 3.91 | **1107** | **1242** | **-135** | **1923** | **-201** | **2015** | 203 | **1229** | 117 |
| 6 | 7.81 | **2320** | **2140** | **180** | **3263** | **23** | **2874** | **272** | **2118** | **230** |
| 7 | 15.63 | **4284** | **3270** | **1015** | **4860** | **642** | **3384** | **375** | **3232** | **316** |
| 8 | 31.25 | **6127** | **4000** | **2127** | **5805** | **1397** | 3447 | **536** | **4005** | **461** |
| 9 | 62.5 | **7901** | 4238 | **3662** | 6068 | **2465** | 3359 | **892** | 4227 | **655** |
| 10 | 125.0 | **10406** | 4339 | **6067** | 6138 | **4069** | 3561 | **2173** | 4346 | **1000** |
| 11 | 250.0 | **17903** | 4670 | **13232** | 6504 | **8958** | 3958 | **10012** | 4623 | **2152** |
| 12 | 500.0 | **29808** | 5613 | **24196** | 7514 | **19719** | 5488 | **15228** | 5435 | **8967** |
| 13 | 1000.0 | 27001 | 2013 | **24988** | 3119 | **21671** | 662 | 16007 | 2002 | **14935** |

As shown in Table 9 above, Comparative Example 4 only measured absorbance S0 when the sample was mixed with the first reagent and measured absorbance S1 after the addition of the second reagent and the end of incubation, and obtained the signal value through the difference between the two. When detecting samples containing high concentrations of the analyte, a hook effect appeared. In contrast, Examples 7 to 10, by separately detecting the signal values of the first-stage reaction after the addition of the first reagent and the signal values of the second-stage reaction after the further addition of the second reagent, allowed for more flexible detection methods (e.g., Example 8 used two wavelengths for detection, and Examples 9 and 10 adjusted the volumes of the reagent and sample added to increase the analyte concentration in the first-stage reaction or decrease the analyte concentration in the second-stage reaction). This improved the sensitivity for detecting low-concentration analyte samples (the SL signal value was significantly increased compared to that of Comparative Example 4) and/or avoided the hook effect when detecting high-concentration analyte samples, thereby expanding the linear detection range.

### Comparative Example 5. Detection of CRP

Reagent R1 (buffer) and reagent R2 (without latex microspheres) were prepared according to the following formulations:
Reagent R1:

| | |
|---|---|
| 50mM Tris buffer | pH 7.5 |
| PEG (PEG6000) | 55g/L |

Reagent R2:

| | |
|---|---|
| 50mM Tris buffer | pH 7.5 |
| CRP antibody | 10g/L |

The samples were detected using the sample analyzer. The specific method was as follows: 200 µL of reagent R1 was added to the reaction cup, and then 8 µL of the sample was added, mixed, and incubated at 37°C for 4 minutes. Then, the absorbance S0 was read at a wavelength of 340 nm. 60 µL of reagent R2 was added to the reaction cup, mixed well, and incubated at 37°C for 4 minutes, and then absorbance S1 was read at a wavelength of 340 nm.

The signal value S was obtained based on the absorbance readings: $Signal value S = S 1 - S 0$

The results are shown in Table 10.

### Comparative Example 6. Detection of CRP

Reagent R1 (buffer) and reagent R2 containing latex microspheres of two particle sizes were prepared according to the following formulations:
Reagent R1:

| | |
|---|---|
| 20mM phosphate buffer | pH 7.5 |
| PEG (PEG6000) | 55g/L |

Reagent R2:

| | |
|---|---|
| 20mM phosphate buffer | pH 7.5 |
| 190nm latex microspheres conjugated with CRP antibody | 100mg/L |
| 70nm latex microspheres conjugated with CRP antibody | 1000mg/L |

The samples were detected using the sample analyzer. The specific method was as follows: 100 µL of reagent R1 was added to the reaction cup, followed by the addition of 2 µL of the sample, mixed, and incubated at 37°C for 4 minutes. 100 µL of reagent R2 was added to the reaction cup and mixed well, and immediately at 0.5 minutes after the addition was complete, absorbance S0 was read at a wavelength of 570 nm. After the incubation was performed at 37°C for 5 minutes, absorbance S1 was read at a wavelength of 570 nm.

The signal value S was obtained based on the absorbance readings above: $Signal value S = S 1 - S 0 .$

The results are shown in Table 10.

### Example 11. Detection of CRP

Reagent R1 (i.e., the first reagent) and reagent R2 (i.e., the second reagent) without latex microspheres were prepared according to the following formulations:
Reagent R1:

| | |
|---|---|
| 20mM phosphate buffer | pH 7.5 |
| PEG (PEG6000) | 15g/L |
| 100nm latex microspheres conjugated with CRP antibody | 150mg/L |

Reagent R2:

| | |
|---|---|
| 20mM phosphate buffer | pH 7.5 |
| CRP antibody | 10g/L |

The samples were detected using the sample analyzer. The specific method was as follows: 150 µL of reagent R1 was added to the reaction cup, followed by the addition of 10 µL of the sample and mixed, and immediately at 0.5 minutes after the addition was complete, blank absorbance S0L was read at a wavelength of 570 nm. After the incubation was performed at 37°C for 4 minutes, absorbance S1L was read at a wavelength of 570 nm, and absorbance S0H was simultaneously read at a wavelength of 340 nm. Then, 50 µL of reagent R2 was added to the reaction cup, mixed well, and incubated at 37°C for 5 minutes, and absorbance S1H was read at a wavelength of 340 nm.

Based on the above absorbance readings, the low-concentration range signal value SL (i.e., the first reaction analysis value) and the high-concentration range signal value SH (i.e., the second reaction analysis value) were obtained, respectively: $Low - concentration range signal value SL = S 1 L - S 0 L ;$ $High - concentration range signal value SH = S 1 H - S 0 H \times 160 / 210 ,$ wherein, 160/210 was the volume correction factor.

The detection results are shown in Table 10 below.

**Table 10**

| No. | Sample concentration (mg/L) | Comparative Example 5 | Comparative Example 6 | Example 11 | |
|---|---|---|---|---|---|
| | | S | S | SL | SH |
| 1 | 0.0 | -7 | 29 | **10** | 1098 |
| 2 | 0.49 | 3 | 183 | **974** | 941 |
| 3 | 0.98 | 11 | 335 | **1474** | 881 |
| 4 | 1.95 | 35 | 603 | **2304** | 835 |
| 5 | 3.91 | 82 | 1102 | **3416** | 938 |
| 6 | 7.81 | 174 | 1735 | **4375** | 1201 |
| 7 | 15.63 | 347 | 2613 | **4887** | **1561** |
| 8 | 31.25 | 634 | 3774 | 5021 | **1936** |
| 9 | 62.5 | 1002 | 5602 | 4986 | **2244** |
| 10 | 125.0 | 1411 | 8647 | 4730 | **2741** |
| 11 | 250.0 | 1683 | 13382 | 4184 | **4170** |
| 12 | 500.0 | 2001 | 17276 | 3223 | **7494** |
| 13 | 1000.0 | 2095 | 15230 | 2634 | **9599** |

As can be seen from the table above, Comparative Example 5, using a non-latex-enhanced immunoturbidimetric method, had poor sensitivity; Comparative Example 6, using latex microspheres of two different particle sizes, showed a significant increase in sensitivity, but a hook effect appeared at high concentrations. The method in Example 11 showed improvements in both sensitivity and detection upper limit.

### Example 12. Detection of CRP

Reagent R1 (i.e., the first reagent), reagent R2 (i.e., the second reagent), and reagent R3 (i.e., the third reagent) were prepared according to the following formulation.
Reagent R1:

| | |
|---|---|
| 20mM phosphate buffer | pH 8.0 |
| 100nm latex microspheres conjugated with CRP antibody | 100mg/L |

Reagent R2:

| | |
|---|---|
| 50mM phosphate buffer | pH 7.5 |
| CRP antibody | 10g/L |

Reagent R3:

| | |
|---|---|
| 50mM phosphate buffer | pH 7.0 |
| PEG (PEG6000) | 55g/L |
| Blocker (rabbit IgG) | 1g/L |

The samples were detected using the sample analyzer. The specific method was as follows: 160 µL of reagent R3 was added to the reaction cup, followed by the addition of 10 µL of the sample, mixed, and incubated at 37°C for 4 minutes. Then, 50 µL of reagent R1 was added and mixed, and immediately at 0.5 minutes after the addition was complete, blank absorbance S0L (i.e., the first optical signal) was read at a wavelength of 340nm. After the incubation was continued at 37°C for 5 minutes, absorbance S1L (i.e., the second optical signal) was read at a wavelength of 340nm. 50 µL of reagent R2 was added to the reaction cup, mixed well, and incubated at 37°C for 4 minutes, and then absorbance S1H (i.e., the fourth optical signal) was read at a wavelength of 340 nm.

Based on the above absorbance readings, the low-concentration range signal value SL (i.e., the first reaction analysis value) and the high-concentration range signal value SH (i.e., the second reaction analysis value) were obtained, respectively: $Low - concentration range signal value SL = S 1 L - S 0 L ;$ $High - concentration range signal value SH = S 1 H - S 0 L \times 220 / 270 ,$ wherein, 220/270 was the volume correction factor.

The detection results are shown in Table 11 below.

### Example 13. Detection of CRP

Reagent R1 (i.e., the first reagent), reagent R2 (i.e., the second reagent), and reagent R3 (i.e., the third reagent) were prepared according to the same formulations as in Example 12.

The samples were detected using the sample analyzer. The specific method was as follows: 80 µL of reagent R1 was added to the reaction cup, and blank absorbance S0L (i.e., the first optical signal) was read at a wavelength of 570 nm. Then, 10 µL of the sample was added to the reaction cup, mixed well, and incubated at 37°C for 4 minutes, and absorbance S1L (i.e., the second optical signal) was read at a wavelength of 570 nm. Next, 130 µL of reagent R3 was added to the reaction cup, mixed well, and incubated at 37°C for 5 minutes, and absorbance S0H (i.e., the third optical signal) was read at a wavelength of 340 nm. Finally, 50 µL of reagent R2 was added to the reaction cup, mixed well, and incubated at 37°C for 4 minutes, and absorbance S1H (i.e., the fourth optical signal) was read at a wavelength of 340 nm.

Based on the above absorbance readings, the low-concentration range signal value SL (i.e., the first reaction analysis value) and the high-concentration range signal value SH (i.e., the second reaction analysis value) were obtained, respectively: $Low - concentration range signal value SL = S 1 L - S 0 L \times 80 / 90 ;$ $High - concentration range signal value SH = S 1 H - S 0 L \times 220 / 270 ;$ wherein, 80/90 and 220/270 were volume correction coefficients.

The detection results are shown in Table 11 below.

### Example 14. Detection of CRP

Reagent R1 (i.e., the first reagent), reagent R2 (i.e., the second reagent), and reagent R3 (i.e., the third reagent) were prepared according to the following formulations.
Reagent R1:

| | |
|---|---|
| 50mM Tris buffer | pH 7.5 |
| 210nm latex microspheres conjugated with CRP antibody | 60mg/L |

Reagent R2:

| | |
|---|---|
| 50mM Tris buffer | pH 7.5 |
| 60nm latex microspheres conjugated with CRP antibody | 500mg/L |

Reagent R3:

| | |
|---|---|
| 50mM Tris buffer | pH 7.5 |
| CRP antibody | 5g/L |

The samples were detected using the sample analyzer. The specific method was as follows: 120 µL of reagent R1 and 10 µL of the sample were added to the reaction cup, and mixed well, and immediately at 0.5 minutes after the addition was complete, blank absorbance S0L (i.e., the first optical signal) was read at a wavelength of 570 nm. After the incubation was performed at 37°C for 4 minutes, absorbance S1L (i.e., the second optical signal) was read at a wavelength of 570 nm. Then, 40 µL of reagent R2 was added and mixed well, and immediately at 0.5 minutes after the addition, blank absorbance S0M (i.e., the third optical signal) was read at a wavelength of 570 nm. The incubation was continued at 37°C for 5 minutes, and then absorbance S1M (i.e., the fourth optical signal) was read at a wavelength of 570 nm, and absorbance S0H (i.e., the fifth optical signal) was simultaneously read at a wavelength of 340 nm. Finally, 50 µL of reagent R3 was added to the reaction cup, mixed well, and incubated at 37°C for 4 minutes, and absorbance S1H (i.e., the sixth optical signal) was read at a wavelength of 340 nm.

Based on the absorbance readings above, the low-concentration range signal value SL (i.e., the first reaction analysis value), the medium-concentration range signal value SM (i.e., the second reaction analysis value), and the high-concentration range signal value SH (i.e., the third reaction analysis value) were obtained, respectively: $Low - concentration range signal value SL = S 1 L - S 0 L ;$ $Medium - concentration range signal value SM = S 1 M - S 0 M ;$ $High - concentration range signal value SH = S 1 H - S 0 H \times 170 / 220 ;$ wherein, 170/220 was volume correction factor.

The detection results are shown in Table 11 below.

**Table 11**

| No. | Sample concentration (mg/L) | Example 7 | | Example 8 | | Example 9 | | |
|---|---|---|---|---|---|---|---|---|
| | | SL | SH | SL | SH | SL | SM | SH |
| 1 | 0.0 | **-20** | 1344 | **-56** | 963 | **-146** | -78 | 300 |
| 2 | 0.49 | **237** | 1320 | **405** | 966 | **476** | -14 | 275 |
| 3 | 0.98 | **393** | 1359 | **649** | 972 | **941** | 53 | 204 |
| 4 | 1.95 | **566** | **1514** | **946** | **1038** | **1678** | **153** | 132 |
| 5 | 3.91 | **605** | **1851** | **1331** | **1268** | **2526** | **277** | 0 |
| 6 | 7.81 | 506 | **2175** | 1424 | **1583** | **2935** | **457** | -54 |
| 7 | 15.63 | 436 | **2601** | 1336 | **1880** | 2927 | **775** | -120 |
| 8 | 31.25 | 390 | **3372** | 1254 | **2426** | 2846 | **1648** | -325 |
| 9 | 62.5 | 350 | **4665** | 1200 | **3786** | 2910 | **6071** | -1473 |
| 10 | 125.0 | 354 | **6532** | 1095 | **6057** | 3016 | **17160** | **1783** |
| 11 | 250.0 | 327 | **9193** | 942 | **8912** | 3294 | 15001 | **4718** |
| 12 | 500.0 | 317 | **12746** | 531 | **11173** | 5057 | 13653 | **8178** |
| 13 | 1000.0 | 296 | **16992** | 421 | **15327** | 895 | 10054 | **11657** |

As shown in Table 11 above, using three kinds of reagents allowed for more flexible sample detection, thereby achieving effects such as eliminating interference, diluting samples, and further broadening the detection limit.

### Comparative Example 7. Detection of RF using non-latex-enhanced method

The rheumatoid factor (RF II) assay kit (immunoturbidimetric method) 105-004670-00 of Mindary Co., Ltd. was used to detect the samples on the sample analyzer. The specific method was as follows: 180 µL of reagent R1 was added to the reaction cup, followed by the addition of 6 µL of the sample, and incubated at 37°C for 4 minutes, and absorbance S0 was read at a wavelength of 340 nm. Then, 36 µL of reagent R2 was added to the reaction cup, and incubated at 37°C for 4 minutes, and absorbance S1 was read at a wavelength of 340 nm. $Signal value S = S 1 - S 0 \times 186 / 222$

The detection results are shown in Table 12 below.

### Comparative Example 8. Detection of RF using latex-enhanced method

The rheumatoid factor (RF) assay kit (latex-enhanced immunoturbidimetric method) 105-001546-00 of Mindary Co., Ltd. was used to detect the samples on the sample analyzer. The specific method was as follows: 120 µL of reagent R1 was added to the reaction cup, followed by the addition of 4 µL of the sample, and incubated at 37°C for 4 minutes. Next, 40 µL of reagent R2 was added to the reaction cup, and absorbance S0 was read at a wavelength of 570 nm. After the incubation was performed at 37°C for 4 minutes, absorbance S1 was read at a wavelength of 570 nm. $Signal value S = S 1 - S 0$

The detection results are shown in Table 12 below.

### Example 15. Detection of RF using two-reagent method

Reagent R1 (i.e., the first reagent) and reagent R2 (i.e., the second reagent) were prepared as follows:
Reagent R1 was prepared by mixing the reagent R1 and reagent R2 from the kit of Comparative Example 8 at a volume ratio of 7:1. Reagent R2 was still the reagent R2 from the kit of Comparative Example 8. The obtained reagent R1 was a reagent containing latex microspheres diluted with the diluent, while the reagent R2 was a undiluted reagent containing latex microspheres.

The samples were detected using the sample analyzer. The specific method was as follows: 120 µL of reagent R1 was added to the reaction cup, and absorbance S0L was read at a wavelength of 380 nm. Then, 4 µL of the sample was added, mixed, and incubated at 37°C for 4 minutes, and absorbance S1L (i.e., the second optical signal) was read at a wavelength of 380 nm. Next, 40 µL of reagent R2 was added, mixed, and incubated at 37°C for 5 minutes, and then absorbance S1H (i.e., the fourth optical signal) was read at a wavelength of 380 nm.

Based on the above absorbance readings, the low-concentration range signal value SL (i.e., the first reaction analysis value) and the high-concentration range signal value SH (i.e., the third reaction analysis value) were obtained, respectively: $Low - concentration range signal value SL = S 1 L - S 0 L \times 120 / 124 ;$ $High - concentration range signal value SH = S 1 H - S 0 H \times 124 / 164 .$
wherein, 120/124 and 124/164 were volume correction coefficients,
and SL + SH was used as the signal value of the output result.

The detection results are shown in Table 12 below.

**Table 12**

| No. | Sample concentration (mg/L) | Comparative Example 7 | Comparative Example 8 | Example 15 | | |
|---|---|---|---|---|---|---|
| | | S | S | SL | SH | SL+SH |
| 1 | 0 | 285 | 625 | 64 | 593 | 657 |
| 2 | 1.17 | 299 | 742 | 128 | 612 | 740 |
| 3 | 2.34 | 325 | **811** | **233** | 595 | **828** |
| 4 | 4.69 | 366 | **1245** | **416** | 618 | **1034** |
| 5 | 9.38 | 435 | **1938** | **861** | 692 | **1553** |
| 6 | 18.75 | 548 | **3782** | **1788** | 733 | **2521** |
| 7 | 37.50 | 719 | **8295** | **3447** | **951** | **4398** |
| 8 | 75.00 | 985 | **11087** | **5301** | **1223** | **6524** |
| 9 | 150.00 | 1413 | 8165 | **6545** | **1487** | **8032** |
| 10 | 300.00 | 2059 | 3249 | **7205** | **1897** | **9102** |
| 11 | 600.00 | 3064 | 631 | **7793** | **2443** | **10236** |
| 12 | 1200.00 | 4148 | -51 | 7630 | **5102** | **12732** |

As shown in Table 12 above, Comparative Example 7, using the non-latex-enhanced method, had lower detection sensitivity, while Comparative Example 8, using the conventional latex-enhanced method, showed a significant hook effect for high-concentration samples. In contrast, Example 15, using the method of the present disclosure, achieved both higher sensitivity and higher detection upper limit.

The above description merely illustrates preferred embodiments of the present disclosure and does not limit the scope of the present disclosure. Any equivalent structural modifications made on the basis of the inventive concept as well as the contents of the description and drawings of the present disclosure, or direct/indirect applications in other related technical fields, all fall into the protection scope of the present disclosure.

## Claims

1. A sample analyzer, comprising: a sample component, a reagent component, a measurement component, and a controller, wherein:
the sample component comprises a sample-carrying component for carrying a sample to be tested and a sample-dispensing mechanism;
the reagent component comprises a reagent-carrying component for carrying a reagent and a reagent-dispensing mechanism;
the measurement component comprises a reaction component and an optical measurement component, wherein the reaction component has at least one placement position for placing a reaction cup;
the controller is configured to perform the following operations:
controlling the reagent-dispensing mechanism to draw a first reagent and dispense the first reagent into the reaction cup, wherein the first reagent comprises a first carrier, the surface of which is bound with a first ligand capable of binding to an analyte in the sample to be tested; controlling the sample-dispensing mechanism to draw the sample to be tested and dispense the sample to be tested into the reaction cup to obtain a first reaction system; controlling the first reaction system to react for a first predetermined time;
controlling the reagent-dispensing mechanism to further draw a second reagent and dispense the second reagent it into the reaction cup to obtain a second reaction system, wherein the second reagent comprises a second ligand capable of binding to the analyte; controlling the second reaction system to react for a second predetermined time;
controlling the optical measurement component to acquire at least two optical signals;
obtaining a first reaction analysis value of the first reaction system based on at least one of the optical signals, and obtaining a second reaction analysis value of the second reaction system based on at least another of the optical signals; and
performing a determination based on the first reaction analysis value and/or the second reaction analysis value, and selecting one of the first reaction analysis value and the second reaction analysis value according to a determination result to obtain an analysis result of the analyte in the sample to be tested, or, selecting the optical signal corresponding to one of the first reaction analysis value and the second reaction analysis value according to a determination result to obtain an analysis result of the analyte in the sample to be tested.

2. A sample analyzer, comprising: a sample component, a reagent component, a measurement component, and a controller, wherein:
the sample component comprises a sample-carrying component for carrying a sample to be tested and a sample-dispensing mechanism;
the reagent component comprises a reagent-carrying component for carrying a reagent and a reagent-dispensing mechanism;
the measurement component comprises a reaction component and an optical measurement component, wherein the reaction component has at least one placement position for placing a reaction cup;
the controller is configured to perform the following operations:
controlling the reagent-dispensing mechanism to draw a first reagent and dispense the first reagent into the reaction cup, wherein the first reagent comprises a first carrier, the surface of which is bound with a first ligand capable of binding to an analyte in the sample to be tested; controlling the sample-dispensing mechanism to draw the sample to be tested and dispense the sample to be tested into the reaction cup to obtain a first reaction system; controlling the first reaction system to react for a first predetermined time;
controlling the reagent-dispensing mechanism to further draw a second reagent and dispense the second reagent into the reaction cup to obtain a second reaction system, wherein the second reagent comprises a second ligand capable of binding to the analyte; controlling the second reaction system to react for a second predetermined time;
controlling the optical measurement component to acquire at least two optical signals;
obtaining a first reaction analysis value of the first reaction system based on at least one of the optical signals, and obtaining a second reaction analysis value of the second reaction system based on at least another of the optical signals; and
obtaining an analysis result of the analyte in the sample to be tested based on a summation result of the first reaction analysis value and the second reaction analysis value.

3. The sample analyzer according to claim 1 or 2, wherein controlling the optical measurement component to acquire at least two optical signals, comprises:
acquiring at least one of the optical signals after controlling the reagent-dispensing mechanism to draw the first reagent and dispense the first reagent to the reaction cup and before controlling the reagent-dispensing mechanism to further draw the second reagent and dispense the second reagent to the reaction cup, and acquiring at least another of the optical signals after controlling the reagent-dispensing mechanism to further draw the second reagent and dispense the second reagent to the reaction cup; or,
acquiring the at least two optical signals after controlling the reagent-dispensing mechanism to further draw the second reagent and dispense the second reagent to the reaction cup.

4. The sample analyzer according to claim 1, wherein performing a determination based on the first reaction analysis value and/or the second reaction analysis value, and selecting one of the first reaction analysis value and the second reaction analysis value according to the determination result to obtain an analysis result of the analyte in the sample to be tested, or selecting the optical signal corresponding to one of the first reaction analysis value and the second reaction analysis value according to the determination result to obtain an analysis result of the analyte in the sample to be tested, comprises:
comparing the first reaction analysis value and the second reaction analysis value, and selecting one of the first reaction analysis value and the second reaction analysis value according to a comparison result to obtain the analysis result of the analyte in the sample to be tested, or selecting the optical signal corresponding to one of the first reaction analysis value and the second reaction analysis value according to a comparison result to obtain the analysis result of the analyte in the sample to be tested;
preferably, comparing the first reaction analysis value and the second reaction analysis value, and selecting a larger one between the first reaction analysis value and the second reaction analysis value to obtain the analysis result of the analyte in the sample to be tested, or selecting the optical signal corresponding to a larger one between the first reaction analysis value and the second reaction analysis value to obtain the analysis result of the analyte in the sample to be tested.

5. The sample analyzer according to claim 1, wherein performing a determination based on the first reaction analysis value and/or the second reaction analysis value, and selecting one of the first reaction analysis value and the second reaction analysis value according to the determination result to obtain an analysis result of the analyte in the sample to be tested, or selecting the optical signal corresponding to one of the first reaction analysis value and the second reaction analysis value according to the determination result to obtain an analysis result of the analyte in the sample to be tested, comprises:
determining whether the first reaction analysis value and/or the second reaction analysis value are within a preset range; and selecting one of the first reaction analysis value and the second reaction analysis value according to the determination result to obtain the analysis result of the analyte in the sample to be tested, or selecting the optical signal corresponding to one of the first reaction analysis value and the second reaction analysis value according to the determination result to obtain the analysis result of the analyte in the sample to be tested.

6. The sample analyzer according to any one of claims 1 to 5, wherein controlling the optical measurement component to acquire at least two optical signals, comprises:
acquiring the at least one of the optical signals at a first detection wavelength, and acquiring the at least another of the optical signals at a second detection wavelength;
preferably, the first detection wavelength and the second detection wavelength are different;
more preferably, the first detection wavelength and the second detection wavelength are selected from 320nm to 860nm.

7. The sample analyzer according to any one of claims 1 to 6, wherein,
controlling the optical measurement component to acquire at least two optical signals, comprises:
acquiring a first optical signal after controlling the reagent-dispensing mechanism to draw the first reagent and dispense the first reagent to the reaction cup, or, acquiring a first optical signal after controlling the reagent-dispensing mechanism to draw the first reagent and dispense the first reagent to the reaction cup and controlling the sample-dispensing mechanism to draw the sample to be tested and dispense the sample to be tested to the reaction cup and before start of the first predetermined time,
acquiring a second optical signal after end of the first predetermined time and before controlling the reagent-dispensing mechanism to further draw the second reagent and dispense the second reagent to the reaction cup;
acquiring a third optical signal after controlling the reagent-dispensing mechanism to further draw the second reagent and dispense the second reagent to the reaction cup and before start of the second predetermined time; and
acquiring a fourth optical signal after end of the second predetermined time; and
obtaining a first reaction analysis value of the first reaction system based on at least one of the optical signals, and obtaining a second reaction analysis value of the second reaction system based on at least another of the optical signals, comprises:
obtaining the first reaction analysis value of the first reaction system based on the first optical signal and the second optical signal, and obtaining the second reaction analysis value of the second reaction system based on the third optical signal and the fourth optical signal.

8. The sample analyzer according to any one of claims 1 to 6, wherein,
controlling the optical measurement component to acquire at least two optical signals, comprises:
acquiring a second optical signal after the end of the first predetermined time and before controlling the reagent-dispensing mechanism to further draw the second reagent and dispense the second reagent to the reaction cup; and
acquiring a fourth optical signal after the end of the second predetermined time; and
obtaining a first reaction analysis value of the first reaction system based on at least one of the optical signals, and obtaining a second reaction analysis value of the second reaction system based on at least another of the optical signals, comprises:
obtaining the first reaction analysis value of the first reaction system based on the second optical signal, and obtaining the second reaction analysis value of the second reaction system based on the fourth optical signal or based on the fourth optical signal and the second optical signal.

9. The sample analyzer according to any one of claims 1 to 6, wherein,
controlling the optical measurement component to acquire at least two optical signals, comprises:
acquiring a third optical signal after controlling the reagent-dispensing mechanism to further draw the second reagent and dispense the second reagent to the reaction cup and before the start of the second predetermined time, and
acquiring a fourth optical signal after the end of the second predetermined time; and
obtaining a first reaction analysis value of the first reaction system based on at least one of the optical signals, and obtaining a second reaction analysis value of the second reaction system based on at least another of the optical signals, comprises:
obtaining the first reaction analysis value of the first reaction system based on the third optical signal, and obtaining the second reaction analysis value of the second reaction system based on the fourth optical signal or based on the fourth optical signal and the third optical signal.

10. The sample analyzer according to any one of claims 1 to 6, wherein,
controlling the optical measurement component to acquire at least two optical signals, comprises:
acquiring a first optical signal after controlling the reagent-dispensing mechanism to draw the first reagent and dispense the first reagent to the reaction cup, or, acquiring a first optical signal after controlling the reagent-dispensing mechanism to draw the first reagent and dispense the first reagent to the reaction cup and controlling the sample-dispensing mechanism to draw the sample to be tested and dispense the sample to be tested to the reaction cup, and before start of the first predetermined time;
acquiring a second optical signal after end of the first predetermined time and before controlling the reagent-dispensing mechanism to further draw the second reagent and dispense the second reagent to the reaction cup; and
acquiring a fourth optical signal after the end of the second predetermined time; and
obtaining a first reaction analysis value of the first reaction system based on at least one of the optical signals, and obtaining a second reaction analysis value of the second reaction system based on at least another of the optical signals, comprises:
obtaining the first reaction analysis value of the first reaction system based on the first optical signal and the second optical signal, and obtaining the second reaction analysis value of the second reaction system based on the first optical signal and the fourth optical signal or based on the second optical signal and the fourth optical signal.

11. The sample analyzer according to any one of claims 1 to 6, wherein,
controlling the optical measurement component to acquire at least two optical signals, comprises:
collecting a plurality of optical signals at least during a period within the start of the first predetermined time to the end of the first predetermined time, thereby acquiring a first reaction curve; and
collecting a plurality of the optical signals at least during a period within the start of the second predetermined time to the end of the second predetermined time, thereby acquiring a second reaction curve; and
obtaining a first reaction analysis value of the first reaction system based on at least one of the optical signals, and obtaining a second reaction analysis value of the second reaction system based on at least another of the optical signals, comprises:
obtaining the first reaction analysis value of the first reaction system based on the first reaction curve, and obtaining the second reaction analysis value of the second reaction system based on the second reaction curve;
preferably, obtaining the first reaction analysis value of the first reaction system based on the first reaction curve or based on a characteristic value of the first reaction curve, and obtaining the second reaction analysis value of the second reaction system based on the second reaction curve or based on a characteristic value of the second reaction curve.

12. A sample analyzer, comprising: a sample component, a reagent component, a measurement component, and a controller, wherein,
the sample component comprises a sample-carrying component for carrying a sample to be tested and a sample-dispensing mechanism;
the reagent component comprises a reagent-carrying component for carrying a reagent and a reagent-dispensing mechanism;
the measurement component comprises a reaction component and an optical measurement component, wherein the reaction component has at least one placement position for placing a reaction cup;
the controller is configured to perform the following operations:
controlling the reagent-dispensing mechanism to draw a first reagent and dispense the first reagent into the reaction cup, wherein the first reagent comprises a first carrier, the surface of which is bound with a first ligand capable of binding to an analyte in the sample to be tested; controlling the sample-dispensing mechanism to draw the sample to be tested and dispense the sample to be tested into the reaction cup to obtain a first reaction system; controlling the first reaction system to react for a first predetermined time;
controlling the optical measurement component to acquire at least one optical signal;
obtaining a first reaction analysis value of the first reaction system based on the at least one optical signal;
performing a determination based on the first reaction analysis value;
when the determination result meets a preset condition, obtaining an analysis result of the analyte in the sample to be tested based on the first reaction analysis value;
when the determination result does not meet the preset condition, controlling the reagent-dispensing mechanism to further draw a second reagent and dispense the second reagent into the reaction cup to obtain a second reaction system, the second reagent comprising a second ligand capable of binding to the analyte; controlling the reaction system to react for a second predetermined time;
controlling the optical measurement component to acquire at least another optical signal;
obtaining a second reaction analysis value of the second reaction system based at least on the at least another optical signal; and,
obtaining an analysis result of the analyte in the sample to be tested based at least on the second reaction analysis value.

13. The sample analyzer according to claim 12, wherein performing a determination based on the first reaction analysis value comprises:
determining whether the first reaction analysis value is within a preset range;
if so, obtaining the analysis result of the analyte in the sample to be tested based on the first reaction analysis value or the optical signal corresponding to the first reaction analysis value;
if not, controlling the reagent-dispensing mechanism to further draw a second reagent and dispense the second reagent to the reaction cup to obtain a second reaction system, the second reagent comprising a second ligand capable of binding with the analyte; controlling the reaction of the second reaction system for a second predetermined time;
controlling the optical measurement component to acquire the at least another optical signal;
obtaining a second reaction analysis value of the second reaction system based at least on the at least another optical signal; and
obtaining an analysis result of the analyte in the sample to be tested based at least on the second reaction analysis value.

14. The sample analyzer according to claim 12 or 13, wherein obtaining an analysis result of the analyte in the sample to be tested based at least on the second reaction analysis value, comprises:
obtaining the analysis result of the analyte in the sample to be tested based on the first reaction analysis value and the second reaction analysis value.

15. The sample analyzer according to claim 14, wherein obtaining the analysis result of the analyte in the sample to be tested based at least on the first reaction analysis value and the second reaction analysis value, comprises:
performing a determination based on the first reaction analysis value and/or the second reaction analysis value; and selecting one of the first reaction analysis value and the second reaction analysis value according to the determination result to obtain the analysis result of the analyte in the sample to be tested, or, selecting the optical signal corresponding to one of the first reaction analysis value and the second reaction analysis value according to the determination result to obtain the analysis result of the analyte in the sample to be tested.

16. The sample analyzer according to claim 14, wherein obtaining the analysis result of the analyte in the sample to be tested based on the first reaction analysis value and the second reaction analysis value, comprises:
obtaining the analysis result of the analyte in the sample to be tested based on a summation result of the first reaction analysis value and the second reaction analysis value.

17. The sample analyzer according to any one of claims 1 to 16, wherein the optical signal comprises a scattered optical signal and/or a transmitted optical signal.

18. A sample analysis method, the method comprising:
controlling a sample to be tested to mix with a first reagent to obtain a first reaction system and allowing the first reaction system to react for a first predetermined time, wherein the first reagent comprises a first carrier, the surface of which is bound with a first ligand capable of binding to an analyte in the sample to be tested;
controlling a second reagent to mix with the first reaction system to obtain a second reaction system, and allowing the second reaction system to react for a second predetermined time, wherein the second reagent comprises a second ligand capable of binding to the analyte;
acquiring at least two optical signals;
obtaining a first reaction analysis value of the first reaction system based on at least one of the optical signals, and obtaining a second reaction analysis value of the second reaction system based on at least another of the optical signals; and
performing a determination based on the first reaction analysis value and/or the second reaction analysis value, selecting one of the first reaction analysis value and the second reaction analysis value according to a determination result to obtain an analysis result of the analyte in the sample to be tested, or selecting the optical signal corresponding to one of the first reaction analysis value and the second reaction analysis value according to a determination result to obtain an analysis result of the analyte in the sample to be tested.

19. A sample analysis method, the method comprising:
controlling a sample to be tested to mix with a first reagent to obtain a first reaction system and allowing the first reaction system to react for a first predetermined time, wherein the first reagent comprises a first carrier, the surface of which is bound with a first ligand capable of binding to an analyte in the sample to be tested;
controlling a second reagent to mix with the first reaction system to obtain a second reaction system, and allowing the second reaction system to react for a second predetermined time, wherein the second reagent comprises a second ligand capable of binding to the analyte;
acquiring at least two optical signals;
obtaining a first reaction analysis value of the first reaction system based on at least one of the optical signals, and obtaining a second reaction analysis value of the second reaction system based on at least another of the optical signals; and
obtaining an analysis result of the analyte in the sample to be tested based on a summation result of the first reaction analysis value and the second reaction analysis value.

20. The sample analysis method according to claim 18 or 19, wherein,
acquiring at least two optical signals comprises:
acquiring at least one of the optical signals after controlling the sample to be tested to mix with the first reagent and before controlling the second reagent to mix with the first reaction system, and acquiring at least another of the optical signals after controlling the second reagent to mix with the first reaction system; or
acquiring the at least two optical signals after controlling the second reagent to mix with the first reaction system.

21. The sample analysis method according to claim 18, wherein performing a determination based on the first reaction analysis value and/or the second reaction analysis value, and selecting one of the first reaction analysis value and the second reaction analysis value according to the determination result to obtain an analysis result of the analyte in the sample to be tested, or selecting the optical signal corresponding to one of the first reaction analysis value and the second reaction analysis value according to the determination result to obtain an analysis result of the analyte in the sample to be tested, comprises:
comparing the first reaction analysis value with the second reaction analysis value, and selecting one of the first reaction analysis value and the second reaction analysis value according to a comparison result to obtain the analysis result of the analyte in the sample to be tested, or selecting the optical signal corresponding to one of the first reaction analysis value and the second reaction analysis value according to a comparison result to obtain the analysis result of the analyte in the sample to be tested;
preferably, comparing the first reaction analysis value with the second reaction analysis value, and selecting a larger one between the first reaction analysis value and the second reaction analysis value according to the comparison result to obtain the analysis result of the analyte in the sample to be tested, or selecting the optical signal corresponding to a larger one between the first reaction analysis value and the second reaction analysis value according to the comparison result to obtain the analysis result of the analyte in the sample to be tested.

22. The sample analysis method according to claim 18, wherein performing a determination based on the first reaction analysis value and/or the second reaction analysis value, and selecting one of the first reaction analysis value and the second reaction analysis value according to the determination result to obtain an analysis result of the analyte in the sample to be tested, or selecting the optical signal corresponding to one of the first reaction analysis value and the second reaction analysis value according to the determination result to obtain an analysis result of the analyte in the sample to be tested, comprises:
determining whether the first reaction analysis value or the second reaction analysis value is within a preset range; and selecting one of the first reaction analysis value and the second reaction analysis value according to the determination result to obtain the analysis result of the analyte in the sample to be tested, or selecting the optical signal corresponding to one of the first reaction analysis value and the second reaction analysis value according to the determination result to obtain the analysis result of the analyte in the sample to be tested.

23. The sample analysis method according to any one of claims 18 to 22, wherein acquiring at least two optical signals comprises:
acquiring the at least one of the optical signals at a first detection wavelength, and acquiring the at least another of the optical signals at a second detection wavelength;
preferably, the first detection wavelength and the second detection wavelength are not the same;
more preferably, the first detection wavelength and the second detection wavelength are selected from 320 nm to 860 nm.

24. The sample analysis method according to any one of claims 18 to 23, wherein,
acquiring at least two optical signals comprises:
acquiring a first optical signal based on the first reagent or acquiring a first optical signal after controlling the sample to be tested to mix with the first reagent and before start of the first predetermined time;
acquiring a second optical signal after end of the first predetermined time and before controlling the second reagent to mix with the first reaction system;
acquiring a third optical signal after controlling the second reagent to mix with the first reaction system and before start of the second predetermined time; and
acquiring a fourth optical signal after end of the second predetermined time; and
obtaining a first reaction analysis value of the first reaction system based on at least one of the optical signals, and obtaining a second reaction analysis value of the second reaction system based on at least one other optical signal, comprises:
obtaining the first reaction analysis value of the first reaction system based on the first optical signal and the second optical signal, and obtaining the second reaction analysis value of the second reaction system based on the third optical signal and the fourth optical signal.

25. The sample analysis method according to any one of claims 18 to 23, wherein,
acquiring at least two optical signals comprises:
acquiring a second optical signal after the end of the first predetermined time and before controlling the second reagent to mix with the first reaction system, and
acquiring a fourth optical signal after the end of the second predetermined time; and
obtaining a first reaction analysis value of the first reaction system based on at least one of the optical signals, and obtaining a second reaction analysis value of the second reaction system based on at least another of the optical signals, comprises:
obtaining the first reaction analysis value of the first reaction system based on the second optical signal, and obtaining the second reaction analysis value of the second reaction system based on the fourth optical signal or based on the fourth optical signal and the second optical signal.

26. The sample analysis method according to any one of claims 18 to 23, wherein,
acquiring at least two optical signals comprises:
acquiring a third optical signal after controlling the second reagent to mix with the first reaction system and before the start of the second predetermined time;
acquiring a fourth optical signal after the end of the second predetermined time; and
obtaining a first reaction analysis value of the first reaction system based on at least one of the optical signals, and obtaining a second reaction analysis value of the second reaction system based on at least another of the optical signals, comprises:
obtaining the first reaction analysis value of the first reaction system based on the third optical signal, and obtaining the second reaction analysis value of the second reaction system based on the fourth optical signal or based on the fourth optical signal and the third optical signal.

27. The sample analysis method according to any one of claims 18 to 23, wherein,
acquiring at least two optical signals comprises:
acquiring a first optical signal based on the first reagent or acquiring a first optical signal after controlling the sample to be tested to mix with the first reagent and before start of the first predetermined time;
acquiring a second optical signal after end of the first predetermined time and before controlling the second reagent to mix with the first reaction system; and
acquiring a fourth optical signal after the end of the second predetermined time; and
acquiring a first reaction analysis value of the first reaction system based on at least one of the optical signals, and obtaining a second reaction analysis value of the second reaction system based on at least another of the optical signals, comprises:
obtaining the first reaction analysis value of the first reaction system based on the first optical signal and the second optical signal, and obtaining the second reaction analysis value of the second reaction system based on the first optical signal and the fourth optical signal or based on the second optical signal and the fourth optical signal.

28. The sample analysis method according to any one of claims 18 to 23, wherein:
acquiring at least two optical signals comprises:
collecting a plurality of optical signals during at least a period within the start of the first predetermined time to the end of the first predetermined time, thereby acquiring a first reaction curve; and
collecting a plurality of optical signals during at least a period within the start of the second predetermined time to the end of the second predetermined time, thereby acquiring a second reaction curve; and
obtaining a first reaction analysis value of the first reaction system based on at least one of the optical signals, and obtaining a second reaction analysis value of the second reaction system based on at least another of the optical signals, comprises:
obtaining the first reaction analysis value of the first reaction system based on the first reaction curve, and obtaining the first reaction analysis value of the first reaction system based on the second reaction curve;
preferably, obtaining the first reaction analysis value of the first reaction system based on the first reaction curve or based on a characteristic value of the first reaction curve, and obtaining the first reaction analysis value of the first reaction system based on the second reaction curve or based on a characteristic value of the second reaction curve.

29. The sample analysis method according to any one of claims 18 to 28, wherein a binding affinity of the first ligand to the analyte is higher than a binding affinity of the second ligand to the analyte.

30. The sample analysis method according to any one of claims 18 to 29, wherein the second ligand in the second reagent exists in a free form and/or in a form bound to the surface of a second carrier.

31. The sample analysis method according to claim 30, wherein the first carrier in the first reagent comprises latex microspheres having a first particle size, the second carrier in the second reagent comprises latex microspheres having a second particle size, and the second particle size is smaller than the first particle size.

32. The sample analysis method according to any one of claims 18 to 31, wherein the first ligand and the second ligand are antigens or antibodies capable of binding to the analyte.

33. The sample analysis method according to any one of claims 18 to 32, wherein the optical signal comprises a scattered optical signal and/or a transmitted optical signal.

34. A kit, comprising a first reagent and a second reagent, wherein:
the first reagent comprises a first carrier with a first ligand bound to a surface of the first carrier;
the second reagent comprises a second ligand in a free form; and
the first ligand and the second ligand are same or different, and are capable of binding to a same analyte in the same sample.

35. The kit according to claim 34, wherein the first carrier comprises first latex microspheres, and the first latex microspheres have a particle size of 40 nm to 500 nm, preferably 80 nm to 400 nm.

36. The kit according to claim 34 or 35, wherein the first ligand and the second ligand are antigens or antibodies capable of binding to an analyte in the sample to be tested.

37. The kit according to any one of claims 34 to 36, wherein the binding affinity of the first ligand to the analyte is higher than the binding affinity of the second ligand to the analyte.

38. The kit according to any one of claims 34 to 37, wherein the first reagent further comprises a first buffer and an optional anti-interference agent; the second reagent further comprises a second buffer.

39. The kit according to any one of claims 34 to 38, further comprising a pretreatment reagent, wherein the pretreatment reagent comprises a third buffer and an optional anti-interference agent.
